(19) Europäisches Patentamt
European Patent Office
Office européen des brevets

(11) **EP 2 517 614 B1**

(12) **EUROPEAN PATENT SPECIFICATION**

(45) Date of publication and mention
of the grant of the patent:
**30.08.2017 Bulletin 2017/35**

(51) Int Cl.:
***A61B 1/00*** *(2006.01)* ***G01N 21/27*** *(2006.01)*

(21) Application number: **11739628.3**

(22) Date of filing: **20.01.2011**

(86) International application number:
**PCT/JP2011/050951**

(87) International publication number:
**WO 2011/096279 (11.08.2011 Gazette 2011/32)**

(54) **IMAGE PROCESSING DEVICE, ENDOSCOPE SYSTEM, PROGRAM AND IMAGE PROCESSING METHOD**

BILDVERARBEITUNGSVORRICHTUNG, ENDOSKOPSYSTEM, PROGRAMM UND BILDVERARBEITUNGSVERFAHREN

DISPOSITIF DE TRAITEMENT D'IMAGES, SYSTÈME D'ENDOSCOPE, PROGRAMME ET PROCÉDÉ DE TRAITEMENT D'IMAGES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.02.2010 JP 2010023750**

(43) Date of publication of application:
**31.10.2012 Bulletin 2012/44**

(73) Proprietor: **Olympus Corporation
Tokyo 192-8507 (JP)**

(72) Inventor: **TSURUOKA, Takao
Hachioji-shi,
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(56) References cited:
**EP-A1- 1 839 558     JP-A- 2002 095 635
JP-A- 2005 007 145     JP-A- 2007 505 645
US-A1- 2005 059 894**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 517 614 B1

**Description**

TECHNICAL FIELD

The present invention relates to an image processing device, an endoscope system, a program, an image processing method, and the like.

BACKGROUND ART

In the fields of endoscopes, microscopes, and the like, a special light image obtained using special light having specific spectral characteristics has been used in addition to a normal light image obtained using normal white light.

For example, Patent Document 1 discloses an endoscope apparatus that alternately acquires a normal light image obtained using normal white light and a fluorescent image obtained using given excitation light from an object to which a fluorescent substance has been administered, and stores the normal light image and the fluorescent image in a storage device to simultaneously display the normal light image and the fluorescent image. According to the technology disclosed in Patent Document 1, it is possible to improve the capability to specify an attention area (e.g., lesion area) within the normal light image.

Patent Document 2 discloses an endoscope apparatus that alternately acquires a normal light image obtained using normal white light and a special light image obtained using special light having a specific wavelength, stores the normal light image and the special light image in a storage device, subjects the normal light image and the special light image to different image processing, and displays the normal light image and the special light image either independently or in a blended state. According to the technology disclosed in Patent Document 2, it is possible to obtain an optimum normal light image and special light image, and improve the capability to specify an attention area (e.g., lesion area) within the normal light image.

RELATED-ART DOCUMENT

PATENT DOCUMENT

Patent Document 1: JP-A-63-122421
Patent Document 2: JP-A-2004-321244

Document US 2005/0059894 A1 discloses a device according to the preamble of claim 1.

SUMMARY OF THE INVENTION

TECHNICAL PROBLEM

According to the technology disclosed in Patent Documents 1 and 2, the normal light image and the special light image can be displayed simultaneously. However, the capability to specify an attention area (e.g., lesion area) depends on the user (e.g., doctor). Therefore, the user may not be able to specify an attention area when an attention area detected within the special light image moves quickly when capturing an image in a moving state using an endoscope or the like. The user may also not be able to specify an attention area when the area of an attention area detected within the special light image is small even when capturing an image in a stationary state.

According to the technology disclosed in Patent Documents 1 and 2, since the normal light image and the special light image are alternately acquired in an equal ratio, the temporal resolution of the normal light image that is basically used when observing a moving image may become insufficient (i.e., observation may become difficult).

Several aspects of the invention may provide an image processing device, an endoscope system, a program, an image processing method, and the like that prevent a situation in which an attention area is missed, and make it possible to reliably specify an attention area.

Several aspects of the invention may provide an image processing device, an endoscope system, a program, an image processing method, and the like that can suppress a decrease in temporal resolution of a first image that is basically used when observing a moving image, and acquire a high-quality display image.

SOLUTION TO PROBLEM

According to one aspect of the invention, there is provided an image processing device as defined by appended claim 1.

BRIEF DESCRIPTION OF DRAWINGS

FIGS. 1A and 1B illustrate a related-art method.
FIG. 2 is a view illustrating a method that changes the display state of a display image based on the attention area detection result.
FIG. 3 is a view illustrating a designated elapsed time setting method according to one embodiment of the invention.
FIG. 4 is a view illustrating a method that resets a designated elapsed time when an attention area has been detected.
FIG. 5 illustrates a first configuration example according to one embodiment of the invention.
FIGS. 6A and 6B are views illustrating a color filter of a CCD.
FIGS. 7A and 7B are views illustrating spectral characteristics obtained using illumination light and a rotary filter.
FIG. 8 is a view illustrating a cycle period in which a normal light image and a special light image are acquired.
FIGS. 9A and 9B are views illustrating a designated elapsed time setting method depending on detection of an attention area.
FIG. 10 illustrates a configuration example of an attention area detection section.
FIG. 11 is a view illustrating hue/chroma threshold values for detecting an attention area.
FIG. 12 illustrates a configuration example of a designated elapsed time setting section.
FIGS. 13A and 13B are views illustrating a detection information updating method.
FIG. 14 illustrates a configuration example of a display state setting section.
FIG. 15 is a view illustrating an alert information addition method.
FIG. 16 illustrates a configuration example of a computer used for software processing.
FIG. 17 illustrates a configuration example of a computer used for software processing.
FIG. 18 is a flowchart illustrating an overall process according to one embodiment of the invention.
FIG. 19 is a flowchart illustrating an attention area detection process.
FIG. 20 is a flowchart illustrating a designated elapsed time setting process.
FIG. 21 is a flowchart illustrating a display state setting process.
FIG. 22 illustrates a second configuration example according to one embodiment of the invention.
FIG. 23 is a view illustrating a designated elapsed time setting method using a motion amount.
FIG 24 illustrates a configuration example of a designated elapsed time setting section.
FIG. 25 is a view illustrating the relationship between a motion amount and a designated elapsed time.
FIG. 26 illustrates a configuration example of a display state setting section.
FIG. 27 illustrates a third configuration example according to one embodiment of the invention.
FIG. 28 is a view illustrating an illumination light color filter.

DESCRIPTION OF EMBODIMENTS

Exemplary embodiments of the invention are described below. Note that the following exemplary embodiments do not in any way limit the scope of the invention laid out in the claims. Note also that all elements of the following exemplary embodiments should not necessarily be taken as essential elements of the invention.

1. Method

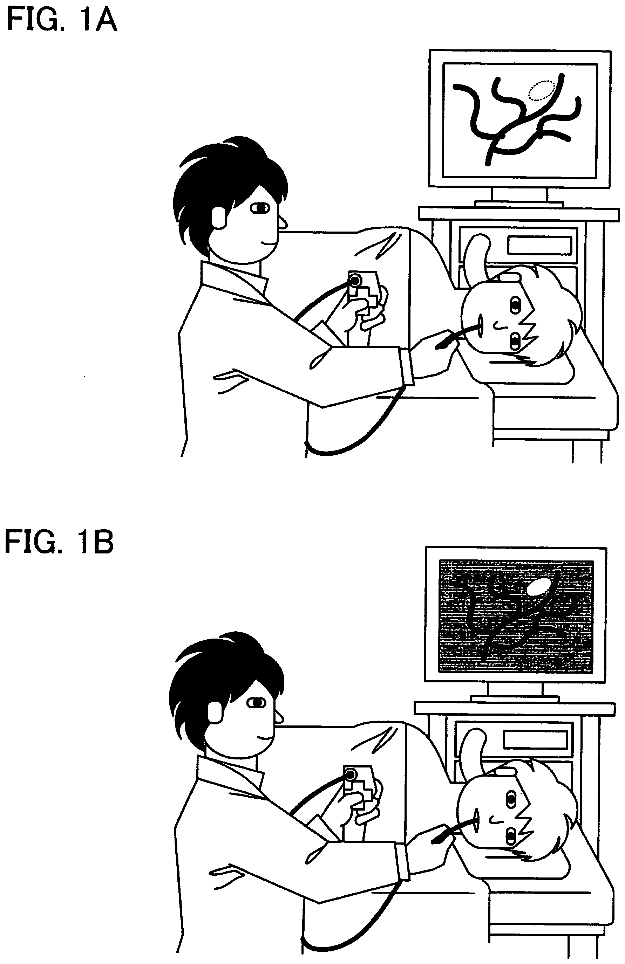

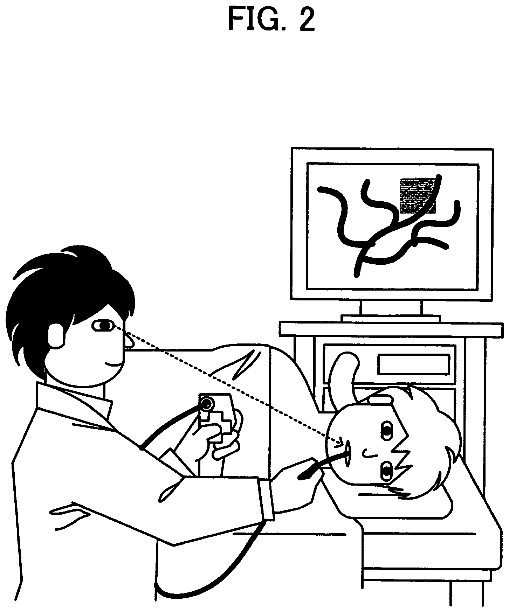

An outline of several embodiments of the invention is described below. FIGS. 1A and 1B illustrate a related-art method. FIG. 1A illustrates the state of observation using normal light. A bright image that can be easily observed is obtained using normal light. However, it is difficult to observe some lesion (e.g., epidermoid cancer) when using normal light. FIG. 1B illustrates the state of observation using special light (e.g., narrow-band light or fluorescence). In this case, the visibility of some lesion can be improved (e.g., a lesion such as epidermoid cancer is displayed in brown) as compared with observation using normal light. However, a dark image that is difficult to observe is obtained using special light.
A method illustrated in FIG 2 may be employed to solve such problems. The method illustrated in FIG. 2 improves the visibility of an attention area (e.g., a lesion area such as epidermoid cancer) by specifying the attention area from the special light image (second image), and processing the normal light image based on the attention area detection result. More specifically, the visibility of the attention area is improved by performing image processing such as blending the color of the normal light image and a given color within the attention area, or enclosing the attention area with a line in a given color.

However, even when employing such a method, the doctor may miss the attention area (e.g., lesion area) when the doctor is paying attention to manipulation of the equipment (see FIG. 2). In particular, an attention area detected within the special light image moves quickly when capturing a moving image using an endoscope.

Several embodiments of the invention employ the following method in order to prevent a situation in which an attention area is missed, and make it possible to reliably specify an attention area.

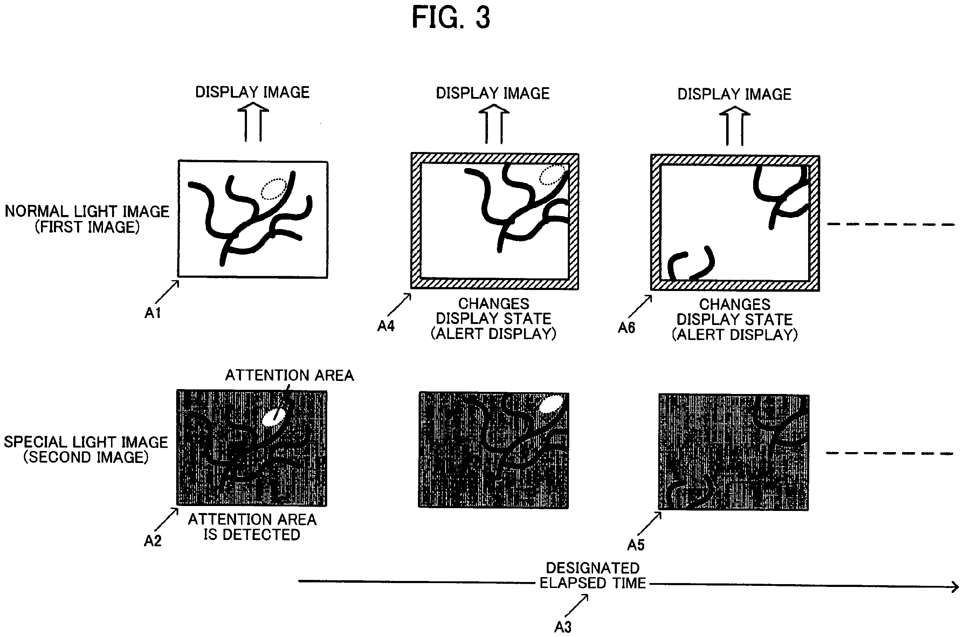

Specifically, a normal light image (first image in a broad sense) and a special light image (second image in a broad sense) are acquired, as indicated by A1 and A2 in FIG 3. The normal light image and the special light image may be acquired in real time using a normal scope endoscope, or may be acquired by reading an image captured using a capsule endoscope or the like from a storage device.

When an attention area has been detected within the special light image (see A2 in FIG. 3), a designated elapsed time in which a display state change process that changes the display state of the display image is performed is set (see A3). The display state change process that changes the display state of the display image (changes the display image) is performed until the designated elapsed time elapses. More specifically, a display image in which alert information about the attention area is set (added, superimposed, or blended) to the normal light image (see A4) is generated, and displayed on a display section until the designated elapsed time elapses. The display state change process is not performed when the designated elapsed time has elapsed, and the normal light image is displayed as the display image, for example.

In FIG. 3, the display state of the display image is continuously changed (see A6) until the designated elapsed time (see A3) elapses even after the attention area has become undetectable within the special light image (see A5). Since the alert information is thus output for a while even after the attention area has become undetectable, it is possible to effectively prevent a situation in which the user (e.g., doctor) misses the attention area.

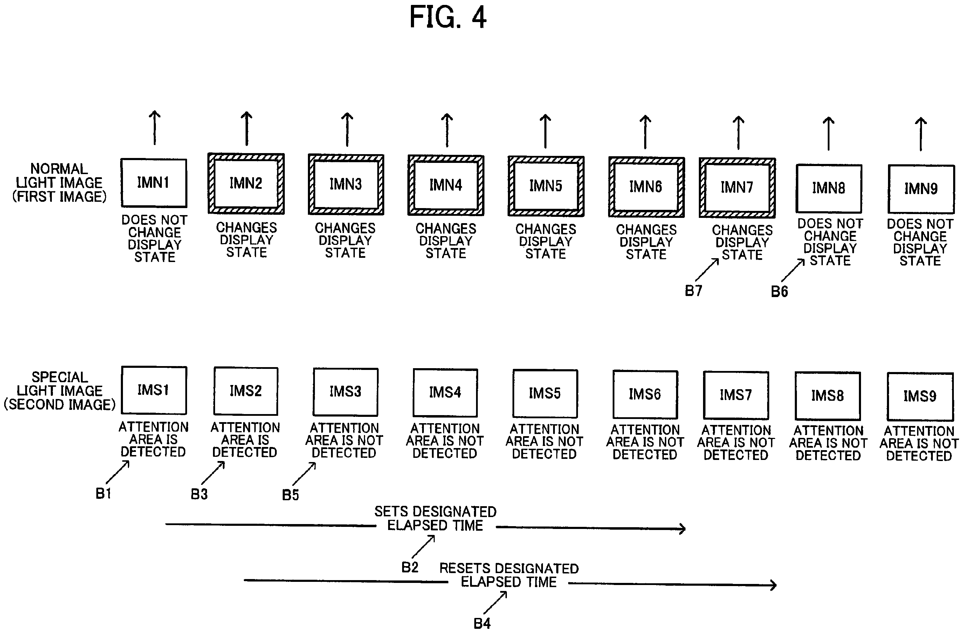

In FIG. 4, normal light images IMN1 to IMN9 and special light images IMS1 to IMS9 are acquired. The normal light images IMN1 to IMN9 are respectively acquired corresponding to the special light images IMS1 to IMS9.

When an attention area (e.g., lesion area) has been detected within the special light image IMS1 (see B1 in FIG 4), a designated elapsed time is set (see B2). The normal light images IMN2 to IMN6 acquired within the designated elapsed time are subjected to the display state change process, and the normal light images IMN2 to INN6 for which the display state is changed are displayed as the display image.

When the attention area has been detected within the special light image IMS2 (see B3) acquired within the designated elapsed time (see B2), the designated elapsed time is reset (see B4). More specifically, a new designated elapsed time that starts from the detection timing of the attention area indicated by B3 is set to extend the designated elapsed time. Therefore, the normal light image IMN7 is also subjected to the display state change process in addition to the normal light images IMN2 to IMN6, and the normal light images IMN2 to IMN7 for which the display state is changed are displayed as the display image. Since the attention area has not been detected within the special light image IMS3 (see B5), the designated elapsed time is not reset, and the normal light image IMN8 (see B6) is not subjected to the display state change process (i.e., the display state of the normal light image IMN8 is not changed).

According to the above method, the normal light image (first image) and the special light image (second image) are acquired, and an attention area is detected based on the feature quantity of each pixel of the special light image, for example. The designated elapsed time setting process that sets the designated elapsed time based on the detection result is performed, and the display state setting process that changes the display state of the display image is performed based on the set designated elapsed time. Therefore, the user can determine that the attention area is present near the current observation position even when the attention area is detected only for a short time during observation in a moving state. This makes it possible to prevent a situation in which the attention area is missed, and reliably specify the attention area. In particular, even after the attention area has become undetectable within the special light image (see A5 in FIG. 3), the display state of the display image is changed (see A6) until the designated elapsed time elapses. Therefore, the alert information is output for a while even if the user (e.g., doctor) has missed the attention area when the user is paying attention to manipulation of the equipment (see FIG. 2). This makes it possible to notify the user of the presence of the attention area, and more reliably specify the attention area.

According to several embodiments of the invention, the normal light image and the special light image are acquired every given cycle period so that the normal light image is acquired in a high ratio as compared with the special light image (details thereof are described later). This makes it possible to prevent a decrease in temporal resolution of the normal light image, and obtain a high-quality normal light image. Moreover, since the alert area is set to the peripheral area of the normal light image, and the alert information (alert image) is set (added) to the alert area (see A4 in FIG 3), it is possible to provide an image processing device that ensures excellent operability without hindering observation of the normal light image.

2. First configuration example

2.1 Overall configuration

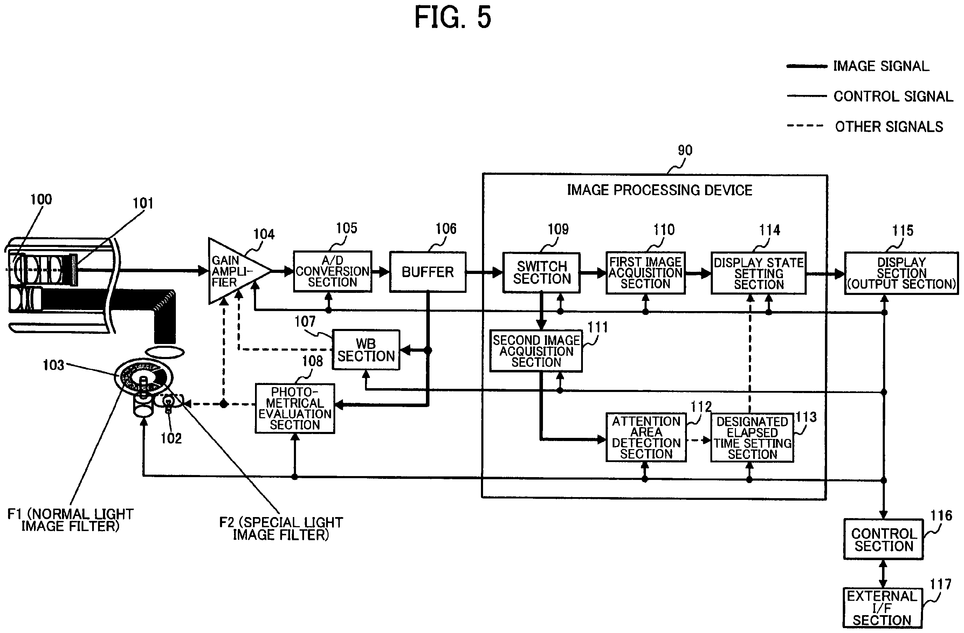

FIG. 5 illustrates a first configuration example according to one embodiment of the invention that implements the above method. FIG. 5 illustrates a configuration example of an image processing device 90 according to one embodiment of the invention and an endoscope system (endoscope apparatus) that includes the image processing device 90. Note that the configuration of the image processing device 90 and the endoscope system is not limited to the configuration illustrated in FIG. 5. Various modifications may be made, such as omitting some of the elements or adding other elements.

An image signal acquired via a lens system 100 (optical system in a broad sense) and a CCD 101 (image sensor in a broad sense) provided on the end (insertion section) of the endoscope is amplified by a gain amplifier 104, and converted into a digital signal by an A/D conversion section 105. Illumination light emitted from an illumination light source 102 passes through a filter (F1 and F2) attached to a rotary filter 103 provided on the end of the endoscope, and is applied to an object via an optical fiber. The digital image signal output from the A/D conversion section 105 is transmitted to a white balance (WB) section 107, a photometrical evaluation section 108, and a switch section 109 through a buffer 106. The WB section 107 is connected to the gain amplifier 104, and the photometrical evaluation section 108 is connected to the illumination light source 102 and the gain amplifier 104.

As illustrated in FIG. 5, the image processing device 90 (image processing section) includes the switch section 109, a first image acquisition section 110, a second image acquisition section 111, an attention area detection section 112, a designated elapsed time setting section 113, and a display state setting section 114. Note that various modifications may be made, such as omitting some of these elements or adding other elements.

The switch section 109 is connected to the first image acquisition section 110 and the second image acquisition section 111. The first image acquisition section 110 is connected to a display section 115 (e.g., liquid crystal display) (output section in a broad sense) through the display state setting section 114. The second image acquisition section 111 is connected to the attention area detection section 112. The attention area detection section 112 is connected to the designated elapsed time setting section 113. The designated elapsed time setting section 113 is connected to the display state setting section 114.

A control section 116 that is implemented by a microcomputer or the like is bidirectionally connected to the rotary filter 103, the gain amplifier 104, the A/D conversion section 105, the WB section 107, the photometrical evaluation section 108, the switch section 109, the first image acquisition section 110, the second image acquisition section 111, the attention area detection section 112, the designated elapsed time setting section 113, the display state setting section 114, and the display section 115. An external I/F (interface) section 117 is bidirectionally connected to the control section 116. The external I/F section 117 includes a power switch, a shutter release button, and an interface for setting (changing) various modes during imaging.

An operation implemented by the first configuration example illustrated in FIG. 5 is described below. The mode is set to an imaging mode when the user has set the imaging conditions using the external I/F section 117, and pressed the shutter release button. The image signal acquired via the lens system 100 and the CCD 101 is successively output as an analog signal at given time intervals. The following description is given taking an example in which the given time interval is 1/30th of a second, and the CCD 101 is a single-chip CCD in which a Bayer primary color filter is disposed on the front side.

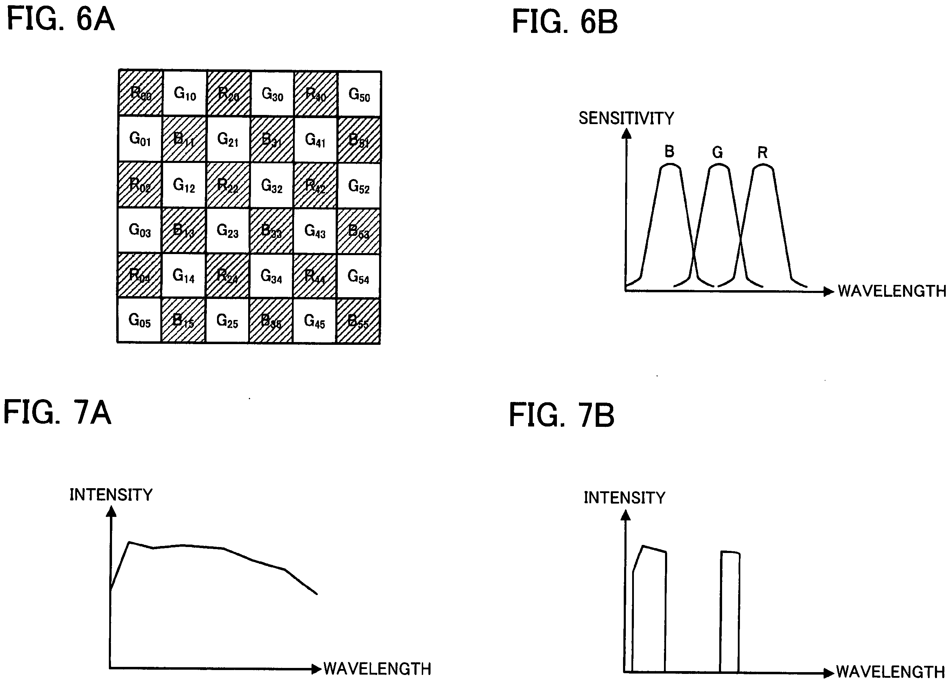

FIG. 6A illustrates a configuration example of the Bayer primary color filter. The basic unit ($2\times2$ pixels) of the Bayer primary color filter includes a red (R) filter (one pixel), a blue (B) filter (one pixel), and a green (G) filter (two pixels). FIG. 6B illustrates the spectral characteristics of the R filter, the G filter, and the B filter.

The following description is given taking an example in which the illumination light source 102 is a normal white light source (e.g., xenon lamp), and two filters are attached to the rotary filter 103. The two filters include a normal light image filter F1 and a special light image filter F2.

FIG. 7A illustrates spectral characteristics obtained when combining the spectral characteristics of illumination light emitted from the illumination light source 102 with the normal light image filter F1. The normal light image filter F1 allows light over the entire visible region to uniformly pass through. The spectral characteristics obtained when combining the spectral characteristics of illumination light emitted from the illumination light source 102 with the normal light image filter F1 are identical with the spectral characteristics of illumination light emitted from the illumination light source 102. An R signal, a G signal, and a B signal of a normal light image are obtained by utilizing the Bayer CCD having the spectral characteristics illustrated in FIG. 6B.

FIG. 7B illustrates spectral characteristics obtained when combining the spectral characteristics of illumination light emitted from the illumination light source 102 with the special light image filter F2. The special light image filter F2 allows only light within a narrow band (390 to 445 nm) of blue light for obtaining information about blood vessels in a surface area and light within a narrow band (530 to 550 nm) of green light for obtaining information about blood vessels in a deep area to pass through (see JP-A-2002-95635, for example). The spectral characteristics obtained when combining the spectral characteristics of illumination light emitted from the illumination light source 102 with the special light image filter F2 consist of the narrow band of blue light and the narrow band of green light. A B signal that corresponds to the narrow band of blue light, a G signal that corresponds to the narrow band of green light, and an R signal that is zero are obtained as signals of a special light image by utilizing the Bayer CCD having the spectral characteristics illustrated in FIG. 6B.

The normal light image filter F1 and the special light image filter F2 are attached to the rotary filter 103 so that the area ratio of the normal light image filter F1 to the special light image filter F2 in the circumferential direction is 29:1. The rotary filter 103 rotates one revolution per second, for example. In this case, a cycle period (T) is 1 second, a period (T1) in which normal light image illumination light is applied is 29/30th of a second, and a period (T2) in which special light image illumination light is applied is 1/30th of a second. Since the image signal is acquired at intervals of 1/30th of a second, twenty-nine normal light images and one special light image are alternately obtained within one cycle period (1 second). Therefore, the temporal resolution of the normal light image is sufficiently maintained.

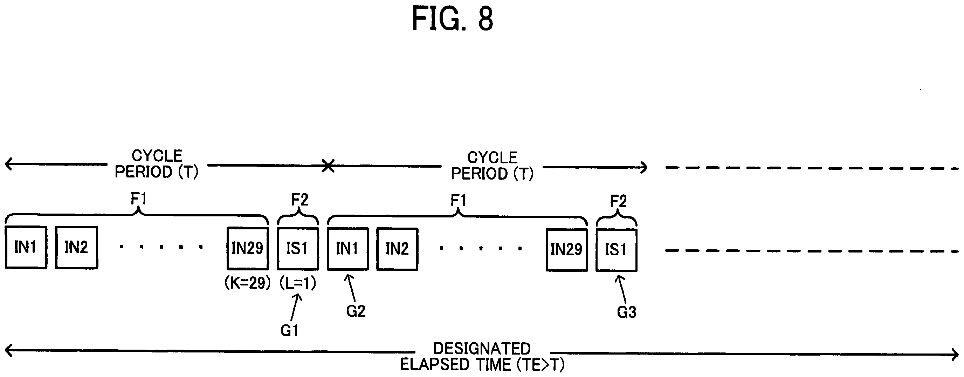

As illustrated in FIG 8, the first image acquisition section 110 acquires a normal light image (first image) corresponding to at least one frame every cycle period (T), for example. More specifically, the first image acquisition section 110 acquires normal light images IN1 to IN29 (K normal light images) corresponding to K frames every cycle period (T). The second image acquisition section 111 acquires a special light image corresponding to at least one frame every cycle period (T). More specifically, the second image acquisition section 111 acquires a special light image IS1 (L special light images) corresponding to L frames every cycle period (T). The normal light images IN1 to IN29 are obtained when applying illumination light to the object through the normal light image filter F1 illustrated in FIG. 5, and the special light image IS1 is obtained when applying illumination light to the object through the special light image filter F2.

In FIG. 8, K=29, and L=1 (i.e., the relationship "K>L" is satisfied). When the relationship "K>L" is satisfied, the number of normal light images acquired within each cycle period (image acquisition period) is larger than the number of special light images acquired within each cycle period. This makes it possible to sufficiently maintain the temporal resolution of the normal light image, and prevent a deterioration in the moving image quality of the display image that may occur when detecting an attention area using the special light image, for example. The special light image is not displayed directly on the display section 115, and the attention area detection process is performed as a background process, for example. Therefore, a serious problem does not occur even if the number L of special light images acquired within each cycle period is small.

When the length of the designated elapsed time set using the method illustrated in FIGS. 3 and 4 is referred to as TE, and the length of the cycle period is referred to as T, the relationship "TE>T" is satisfied (see FIG. 8). This makes it possible to set the length TE of the designated elapsed time to be sufficiently longer than the length T of the cycle period. Therefore, the alert information about the attention area can be displayed for a while until the designated elapsed time elapses (see B7 in FIG. 4) even after the attention area has become undetectable (see B5).

The rotary filter 103 provided on the end of the endoscope is rotated in synchronization with the imaging operation of the CCD 101 under control of the control section 116. The analog image signal obtained by the imaging operation is amplified by the gain amplifier 104 by a given amount, converted into a digital signal by the A/D conversion section 105, and transmitted to the buffer 106. The buffer 106 can store (record) data of one normal light image or one special light image, and a new image acquired by the imaging operation is overwritten.

The normal light image stored in the buffer 106 is intermittently transmitted to the WB section 107 and the photometrical evaluation section 108 at given time intervals under control of the control section 116. The WB section 107 integrates a signal at a given level corresponding to each color signal that corresponds to the color filter to calculate a white balance coefficient. The WB section 107 transmits the white balance coefficient to the gain amplifier 104. The gain amplifier 104 multiplies each color signal by a different gain to implement a white balance adjustment. The photometrical evaluation section 108 controls the intensity of light emitted from the illumination light source 102, the amplification factor of the gain amplifier 104, and the like so that a correct exposure is achieved.

The switch section 109 transmits the normal light image stored in the buffer 106 to the first image acquisition section 110, or transmits the special light image stored in the buffer 106 to the second image acquisition section 111 under control of the control section 116.

The first image acquisition section 110 reads the normal light image from the switch section 109, performs an interpolation process, a grayscale process, and the like on the normal light image, and transmits the resulting normal light image to the display state setting section 114 under control of the control section 116.

The second image acquisition section 111 reads the special light image from the switch section 109, and performs an interpolation process, a grayscale process, and the like on the special light image under control of the control section 116. The second image acquisition section 111 also performs a process that generates a pseudo-color image from the B signal that corresponds to the narrow band of blue light and the G signal that corresponds to the narrow band of green light (see JP-A-2002-95635). The resulting special light image is transmitted to the attention area detection section 112.

The attention area detection section 112 reads the special light image from the second image acquisition section 111, and performs a process that detects a given attention area (e.g., a lesion area in which blood vessels are densely present) under control of the control section 116. The detection result is transmitted to the designated elapsed time setting section 113.

The designated elapsed time setting section 113 reads the attention area detection result from the attention area detection section 112, and determines the designated elapsed time under control of the control section 116, the designated elapsed time being a period of time in which the alert information that indicates information about the detection result is set to the normal light image.

In one embodiment of the invention, the designated elapsed time is set to 5 seconds (five cycle periods) when an attention area has been detected, and the alert information is set for at least 5 seconds even if the attention area has become undetectable, for example. When the attention area has been detected within the designated elapsed time, the designated elapsed time (5 seconds) is reset to start from the detection timing (see B4 in FIG. 4). The designated elapsed time setting section 113 determines whether or not the current time point (current time) is within the set designated elapsed time, and transmits the determination result to the display state setting section 114.

The display state setting section 114 (display state determination section or display control section) reads the determination result as to whether or not the current time point is within the designated elapsed time from the designated elapsed time setting section 113, and selects a process that sets (adds or superimposes) the alert information (alert area) to the normal light image when the current time point is within the designated elapsed time under control of the control section 116. The display state setting section 114 does not perform a process when the current time point is not within the designated elapsed time. Note that the alert information is set to the normal light image acquired after the special light image within which the attention area has been detected. For example, when an attention area has been detected within the special light image IMS1 (see B1 in FIG. 4), the alert information is set (added or superimposed) to the normal light image IMN2 acquired after the special light image IMS1.

The display image generated by the display state setting section 114 is transmitted to the display section 115, and sequentially displayed on the display section 115. The normal light image to which the alert information is set is transmitted as the display image when it has been selected to set the alert information (change the display state), and the normal light image is directly transmitted as the display image when it has been selected not to set the alert information (not to change the display state).

Although FIG. 5 illustrates the display section 115 as an example of the output section, the output section is not limited to the display section 115. The output section may be implemented by sequentially recording (storing) the normal light image in a recording medium such as a hard disk or a memory card. The alert information may be output using sound instead of an image.

According to one embodiment of the invention, the first image acquisition section 110 acquires the normal light image corresponding to at least one frame (K frames) every cycle period (T), and the second image acquisition section 111 acquires the special light image corresponding to at least one frame (L frames) every cycle period (see FIG. 8).

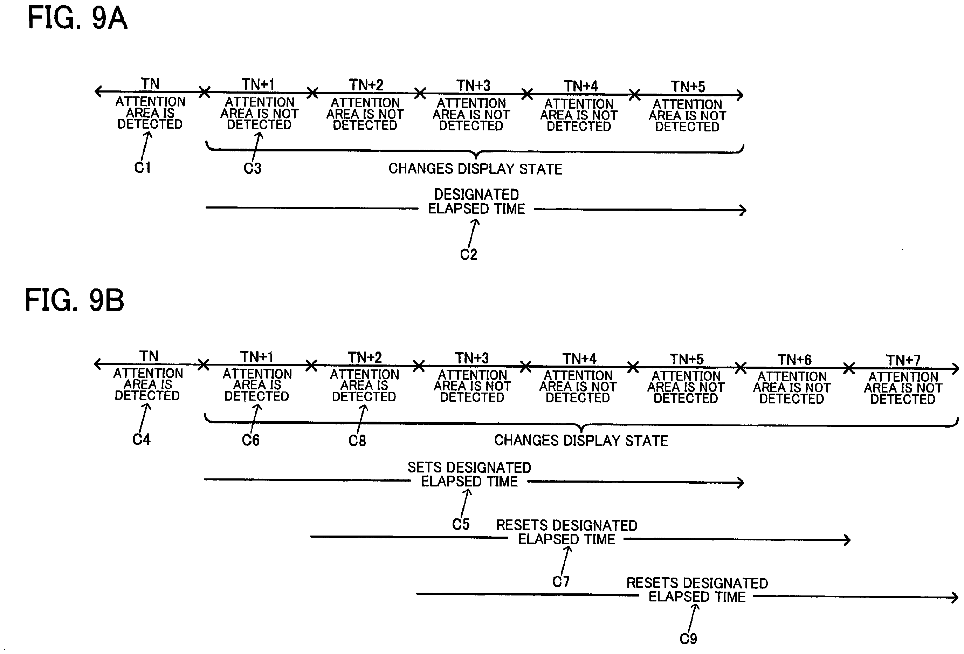

When an attention area has been detected within the special light image in a cycle period TN (Nth cycle period) (see C1 in FIG 9A), the designated elapsed time setting section 113 sets the designated elapsed time (see C2). The display state setting section 114 performs the display state change process in a cycle period TN+1 ((N+1)th cycle period) subsequent to the cycle period TN. For example, when an attention area has been detected within the special light image IS1 (see G1 in FIG. 8), the alert information is set to the normal light image IN1 (see G2) and the subsequent normal light images acquired in the next cycle period (i.e., the display state is changed).

When an attention area has been detected within the special light image in the cycle period TN (see C1 in FIG. 9A), and the attention area has not been detected within the special light image in the cycle period TN+1 (see C3) (e.g., when an attention area has been detected within the special light image IS1 indicated by G1 in FIG. 8, and the attention area has not been detected within the special light image IS1 indicated by G3), the display state setting section 114 changes the display state of the display image in the cycle periods TN+1 to TN+5 ((N+1)th to Mth cycle periods in a broad sense) (see FIG. 9A).

When an attention area has been detected within the special light image in the cycle period TN (see C4 in FIG. 9B), the designated elapsed time has been set (see C5), and the attention area has also been detected within the special light image in the cycle period TN+1 (see C6) (e.g., when an attention area has been detected within the special light image IS1 indicated by G1 in FIG. 8, and the attention area has also been detected within the special light image IS1 indicated by G3), the designated elapsed time is reset (see C7 in FIG. 9B) so that the designated elapsed time is extended. When the attention area has also been detected within the special light image in the cycle period TN+2 (see C8), the designated elapsed time is reset (see C9) so that the designated elapsed time is further extended. Specifically, the display state change process that changes the display state of the display image is performed as long as an attention area is detected, and is continuously performed until the designated elapsed time elapses even after the attention area has become undetectable.

2.2 Attention area detection section

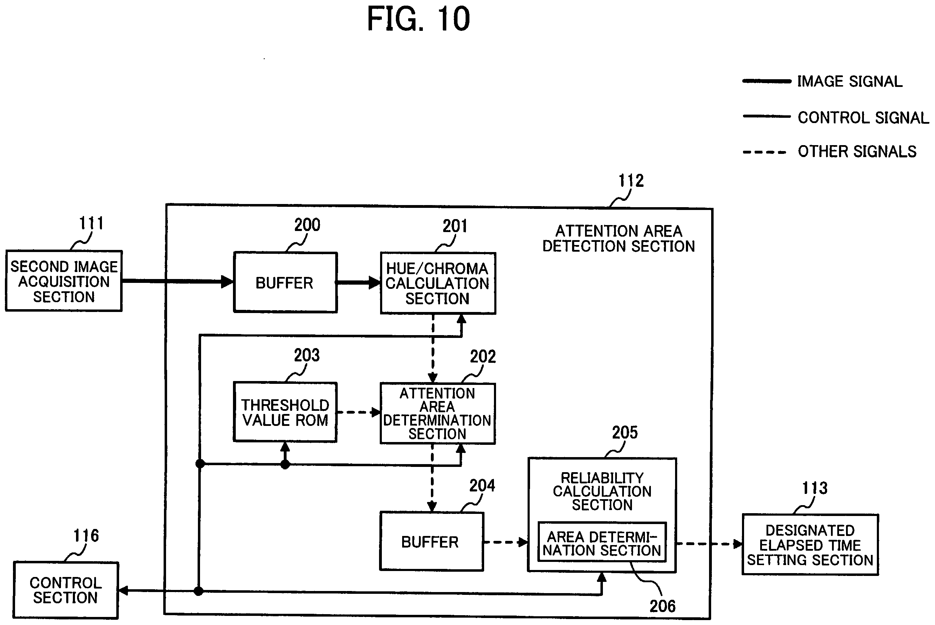

FIG. 10 illustrates a configuration example of the attention area detection section 112. The attention area detection section 112 illustrated in FIG 10 includes a buffer 200, a hue/chroma calculation section 201, an attention area determination section 202, a threshold value ROM 203, a buffer 204, and a reliability calculation section 205. Note that various modifications may be made, such as omitting some of these elements or adding other elements.

As illustrated in FIG. 10, the second image acquisition section 111 is connected to the hue/chroma calculation section 201 via the buffer 200. The hue/chroma calculation section 201 and the threshold value ROM 203 are connected to the attention area determination section 202. The attention area determination section 202 is connected to the reliability calculation section 205 via the buffer 204. The reliability calculation section 205 is connected to the designated elapsed time setting section 113. The control section 116 is bidirectionally connected to the hue/chroma calculation section 201, the attention area determination section 202, the threshold value ROM 203, and the reliability calculation section 205.

The second image acquisition section 111 transmits the special light image (pseudo-color image) to the buffer 200. The hue/chroma calculation section 201 reads the special light image (pseudo-color image) from the buffer 200 under control of the control section 116. The special light image (pseudo-color image) is expressed using an R signal, a G signal, and a B signal. The R signal, the G signal, and the B signal are converted into a luminance signal Y and color difference signals Cb and Cr using the following expressions (1) to (3), for example.

$$Y=0.29900R+0.58700G+0.11400B \qquad (1)$$

$$Cb=-0.16874R-0.33126G+0.50000B \qquad (2)$$

$$Cr=0.50000R-0.41869G-0.08131B \qquad (3)$$

The hue H and the chroma C are calculated using the following expressions (4) and (5).

$$H=\tan^{-1}(Cb/Cr) \qquad (4)$$

$$C=(Cb{\cdot}Cb+Cr{\cdot}Cr)^{1/2} \qquad (5)$$

The hue H and the chroma C thus calculated are sequentially transmitted to the attention area determination section 202 on a pixel basis. The attention area determination section 202 reads the hue H and the chroma C from the hue/chroma calculation section 201, and reads hue threshold values and chroma threshold values from the threshold value ROM 203 under control of the control section 116.

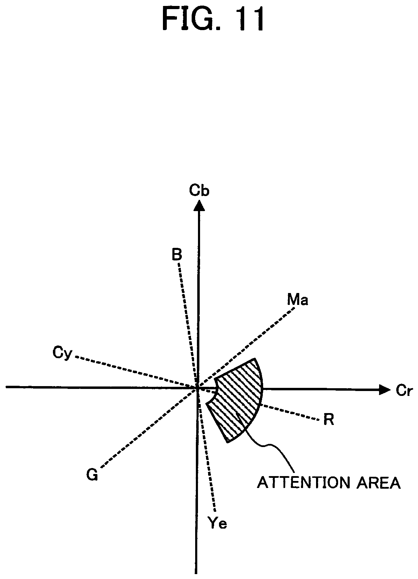

FIG. 11 illustrates an example of threshold values stored in the threshold value ROM 203. In the special light image (pseudo-color image), a surface area (e.g., lesion area) in which blood vessels are densely present is displayed in reddish brown. Therefore, a fan-shaped area illustrated in FIG. 11 corresponds to a lesion area (i.e., attention area). When using an 8-bit signal, the attention area is defined by the following expressions (6) and (7), for example.

$$-70^{o}<\text{hue } H<30^{o} \qquad (6)$$

$$16<\text{chroma } C<128 \qquad (7)$$

The upper-limit value and the lower-limit value of the hue H and the upper-limit value and the lower-limit value of the chroma C (see the expressions (6) and (7)) are stored in the threshold value ROM 203. The attention area determination section 202 reads these four threshold values. The attention area determination section 202 outputs a label value "1" to the buffer 204 corresponding to a pixel that satisfies the expressions (6) and (7), and outputs a label value "0" to the buffer 204 corresponding to a pixel that does not satisfy the expressions (6) and (7). The label value that indicates whether or not each pixel of the special light image belongs to the attention area is thus stored in the buffer 204.

An area determination section 206 included in the reliability calculation section 205 reads the label values from the buffer 204, and calculates the total number of pixels that belong to the attention area to calculate the area of the attention area under control of the control section 116. In one embodiment of the invention, the area of the attention area is used as the reliability that is an index that indicates the likelihood that the attention area is a legion. Specifically, the reliability is calculated based on the area of the attention area. The attention area is determined to have high reliability when the calculated area of the attention area exceeds a given threshold value (i.e., it is determined that an attention area has been detected). For example, it is determined that an attention area has been detected when the calculated area of the attention area exceeds 1% (i.e., threshold value) of the area of the entire image, for example. The attention area is determined to have low reliability when the calculated area of the attention area is equal to or less than the given threshold value (i.e., it is determined that the attention area has not been detected). Detection information that indicates the attention area detection result is transmitted to the designated elapsed time setting section 113.

2.3 Designated elapsed time setting section

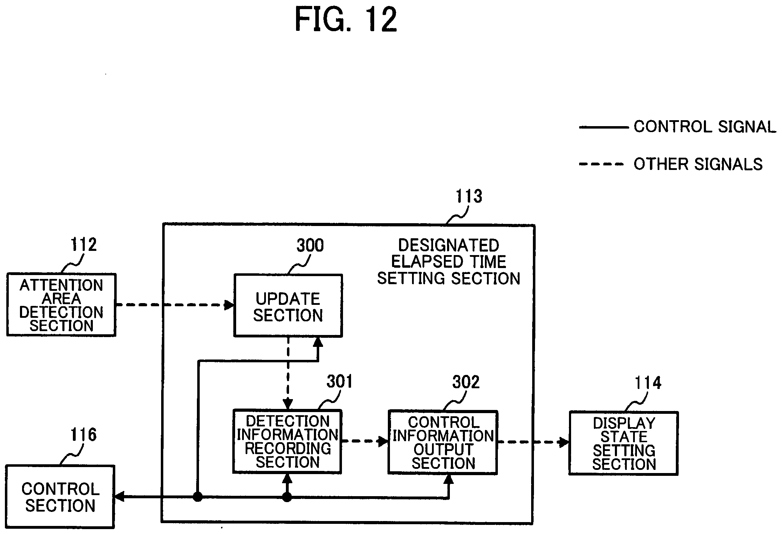

FIG. 12 illustrates a configuration example of the designated elapsed time setting section 113 (designated elapsed time determination section). The designated elapsed time setting section 113 includes an update section 300, a detection information recording section 301, and a control information output section 302 (process control section). Note that various modifications may be made, such as omitting some of these elements or adding other elements.

As illustrated in FIG. 12, the attention area detection section 112 is connected to the control information output section 302 via the update section 300 and the detection information recording section 301. The control information output section 302 is connected to the display state setting section 114. The control section 116 is bidirectionally connected to the update section 300, the detection information recording section 301, and the control information output section 302.

When the image processing device has been initialized upon power on, for example, the control section 116 initializes the detection information recording section 301 to set the value of the detection information to an initial value "0". The update section 300 reads the detection information that indicates whether or not an attention area has been detected within the special light image from the attention area detection section 112 under control of the control section 116.

The update section 300 outputs a given value (e.g., "5") to the detection information recording section 301 as the detection information when an attention area has been detected. The update section 300 decrements the value of the detection information recorded in the detection information recording section 301 by one when an attention area has not been detected. When the value of the detection information has become a negative number, the value of the detection information is set to 0.

The control information output section 302 reads the value of the detection information recorded in the detection information recording section 301 under control of the control section 116. When the value of the detection information is equal to or larger than 1, the control information output section 302 outputs control information that instructs the display state setting section 114 to change the display state (set the alert information) to the display state setting section 114. When the value of the detection information is 0, the control information output section 302 outputs control information that instructs the display state setting section 114 not to change the display state (not to set the alert information) to the display state setting section 114.

In the one embodiment of the invention, the length of the cycle period (T) is 1 second, and one special light image is acquired in each cycle period (see FIG. 8). Therefore, when an attention area has been detected, five cycle periods (i.e., 5 seconds) are required for the value of the detection information to be decremented from "5" to "0". Accordingly, the designated elapsed time in which the display state is changed (i.e., the designated elapsed time in which the alert information is set) is 5 seconds. When the attention area has been detected within the designated elapsed time (5 seconds), the value of the detection information is reset to "5". Therefore, the display state of the display image is changed for 5 seconds from the latest detection timing.

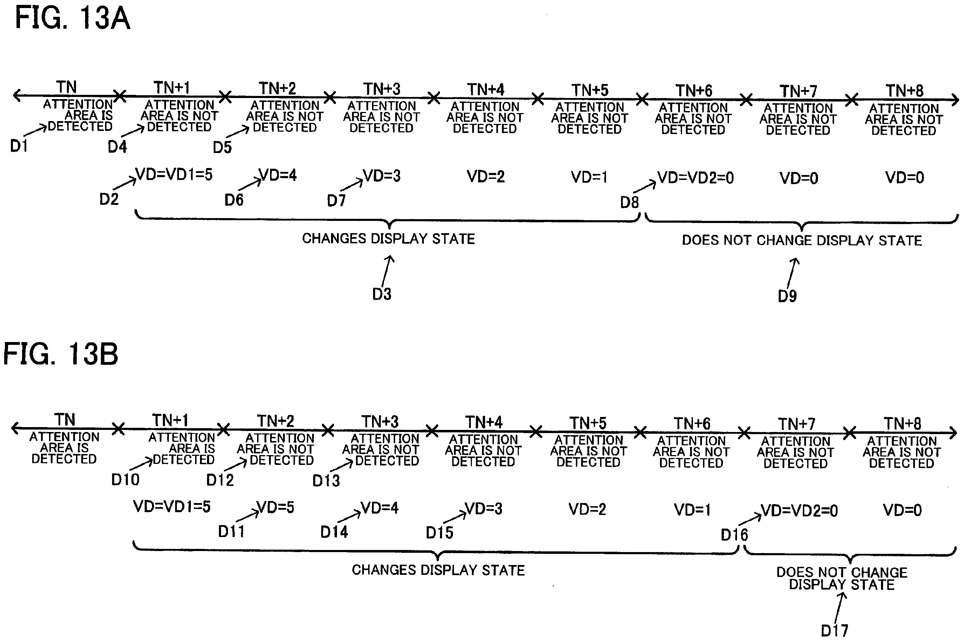

FIGS. 13A and 13B are views illustrating the designated elapsed time setting method according to one embodiment of the invention. In one embodiment of the invention, the detection information recording section 301 records the detection information about the attention area (i.e., information that indicates whether or not an attention area has been detected in each cycle period), and the update section 300 performs an update process that updates the detection information (count value) (count update process) based on the detection result output from the attention area detection section 112. More specifically, the update section 300 performs the update process that updates the detection information every cycle period (T) (see FIG. 8, for example). The control information output section 302 outputs the control information that controls the display state setting process performed by the display state setting section 114 based on the detection information recorded in the detection information recording section 301.

For example, when an attention area has been detected within the special light image in the cycle period TN (see D1 in FIG. 13A), the update section 300 sets the value VD of the detection information to a first value VD1 (=5) that corresponds to the designated elapsed time (see D2). The designated elapsed time (5 seconds) in which the display state change process is performed is set in this manner (see D3).

When the attention area has not been detected within the special light image in the cycle periods TN+1 and TN+2 (see D4 and D5 in FIG. 13A) subsequent to the cycle period TN, the update section 300 performs the update process that sequentially changes the value VD of the detection information from the first value VD1 (=5) to a second value VD2 (=0) (see D6 and D7). Specifically, the update section 300 performs the update process that decrements the value VD of the detection information until the value VD of the detection information reaches the second value VD2 (=0), for example. Note that the update section 300 may perform the update process that increments the value VD of the detection information.

The control information output section 302 outputs the control information that instructs the display state setting section 114 to change the display state to the display state setting section 114 until the value VD of the detection information reaches the second value VD2 (=0). When the value VD of the detection information has reached the second value VD2 (=0) (see D8 in FIG. 13A), the control information output section 302 outputs the control information that instructs the display state setting section 114 not to change the display state to the display state setting section 114 (see D9).

When the attention area has also been detected within the special light image in the cycle period TN+1 (see D10 in FIG. 13B) subsequent to the cycle period TN, the value VD of the detection information is reset to the first value VD1 (=5) (see D11). When the attention area has not been detected within the special light image in the cycle periods TN+2 and TN+3 (see D12 and D13), the update section 300 sequentially changes the value VD of the detection information from the first value VD1 (=5) to the second value VD2 (=0) (see D14 and D15). When the value VD of the detection information has reached the second value VD2 (=0) (see D16), the control information output section 302 instructs the display state setting section 114 not to change the display state (see D 17).

According to one embodiment of the invention, the method illustrated in FIGS. 3 and 4 is implemented by recording the detection information in the detection information recording section 301, and performing the update process that updates the detection information using the method illustrated in FIGS. 13A and 13B. Therefore, the designated elapsed time setting process based on the attention area detection result, and the designated elapsed time reset process performed when the attention area has been detected within the designated elapsed time can be implemented using a simple configuration and a simple process.

2.4 Display state setting section

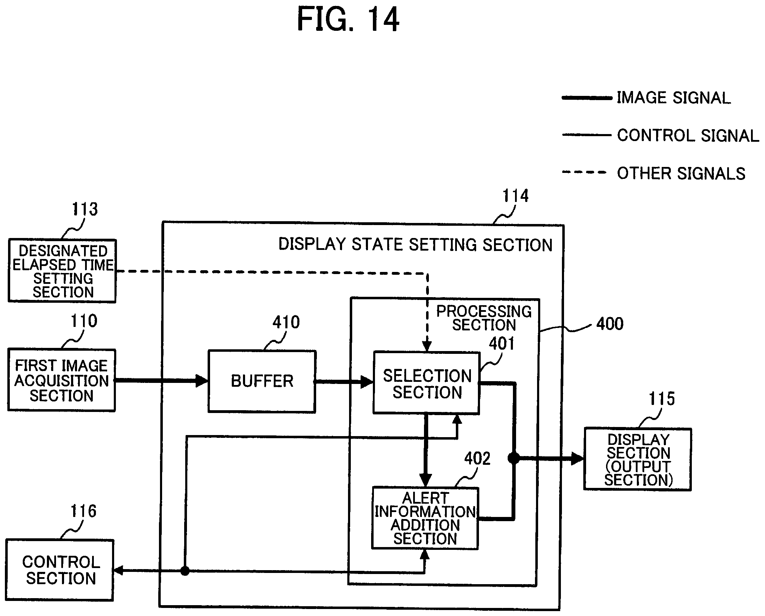

FIG. 14 illustrates a configuration example of the display state setting section 114 (display state determination section). The display state setting section 114 includes a buffer 410 and a processing section 400. The processing section 400 includes a selection section 401 (processing method selection section) and an alert information addition section 402. Note that various modifications may be made, such as omitting some of these elements or adding other elements.

As illustrated in FIG. 14, the first image acquisition section 110 is connected to the selection section 401 via the buffer 410. The designated elapsed time setting section 113 is connected to the selection section 401. The selection section 401 is connected to the alert information addition section 402 and the display section 115. The alert information addition section 402 is connected to the display section 115. The control section 116 is bidirectionally connected to the selection section 401 and the alert information addition section 402.

The normal light image output from the first image acquisition section 110 is transmitted to and stored (recorded) in the buffer 410. The selection section 401 reads the control information that instructs the display state setting section 114 to change or not to change the display state from the designated elapsed time setting section 113 under control of the control section 116. The selection section 401 also reads the normal light image from the buffer 410 under control of the control section 116. When the control information that instructs the display state setting section 114 to change the display state (add the alert information or superimpose the alert area) has been read from the designated elapsed time setting section 113, the selection section 401 transmits the normal light image read from the buffer 410 to the alert information addition section 402. When the control information that instructs the display state setting section 114 not to change the display state has been read from the designated elapsed time setting section 113, the selection section 401 transmits the normal light image read from the buffer 410 to the display section 115.

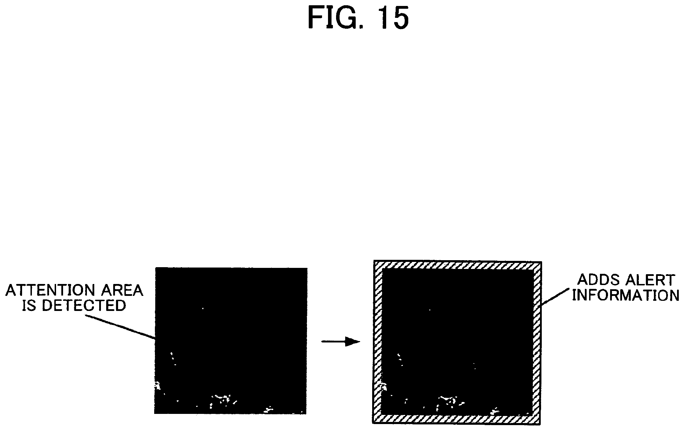

The alert information addition section 402 adds the alert information to the normal light image transmitted from the selection section 401 under control of the control section 116. FIG. 15 illustrates an example of an alert information addition process. In the example illustrated in FIG. 15, an alert color (e.g., red) is superimposed on the alert area (peripheral area) that encloses the normal light image.

2.5 Software processing

Although an example in which each section of the image processing device 90 is implemented by hardware has been described above, the configuration is not limited thereto. For example, a CPU may perform the process of each section on an image acquired using an imaging device such as a capsule endoscope. Specifically, the process of each section may be implemented by software by causing the CPU to execute a program. Alternatively, part of the process of each section may be implemented by software.

When implementing the process of each section of the image processing device 90 by software, a known computer system (e.g., work station or personal computer) may be used as the image processing device. A program (image processing program) that implements the process of each section of the image processing device 90 may be provided in advance, and executed by the CPU of the computer system.

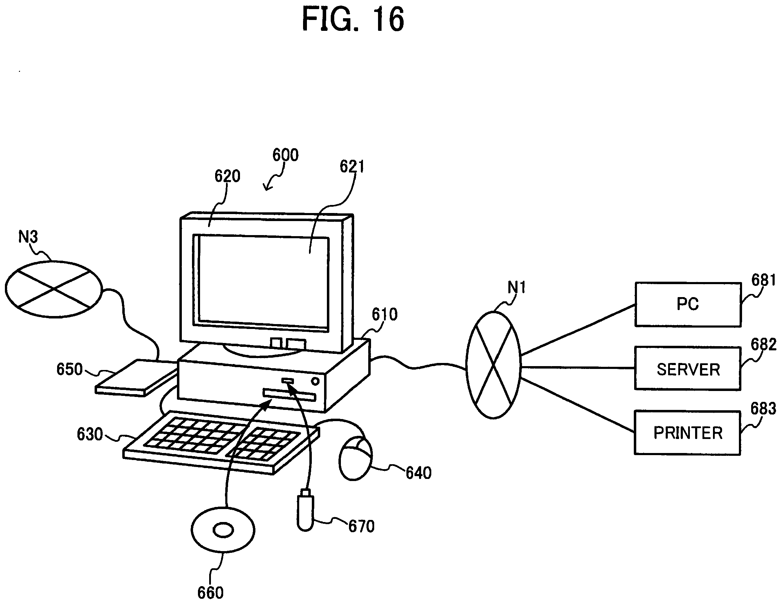

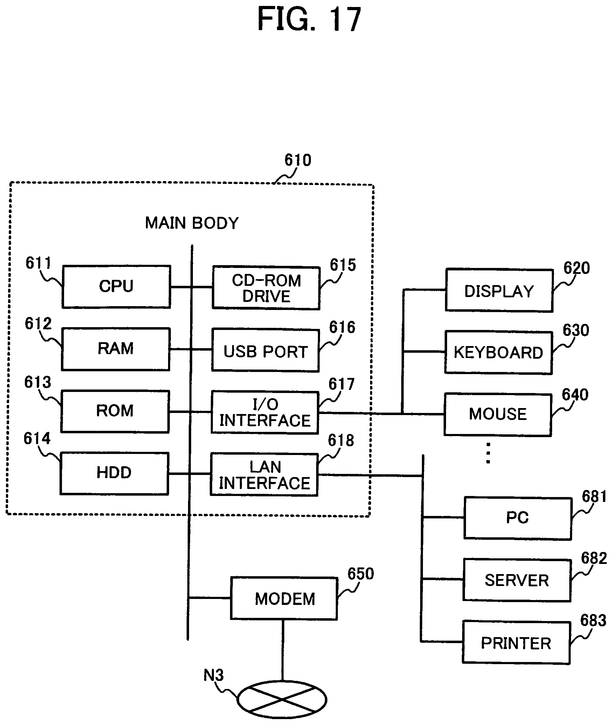

FIG. 16 is a system configuration diagram illustrating the configuration of a computer system 600 according to a modification, and FIG. 17 is a block diagram illustrating the configuration of a main body 610 of the computer system 600. As illustrated in FIG. 16, the computer system 600 includes the main body 610, a display 620 that displays information (e.g., image) on a display screen 621 in accordance with instructions from the main body 610, a keyboard 630 that allows the user to input information to the computer system 600, and a mouse 640 that allows the user to designate an arbitrary position on the display screen 621 of the display 620.

As illustrated in FIG 17, the main body 610 of the computer system 600 includes a CPU 611, a RAM 612, a ROM 613, a hard disk drive (HDD) 614, a CD-ROM drive 615 that receives a CD-ROM 660, a USB port 616 to which a USB memory 670 is removably connected, an I/O interface 617 that connects the display 620, the keyboard 630, and the mouse 640, and a LAN interface 618 that is used to connect to a local area network or a wide area network (LAN/WAN) N1.

The computer system 600 is connected to a modem 650 that is used to connect to a public line N3 (e.g., Internet). The computer system 600 is also connected to a personal computer (PC) 681 (i.e., another computer system), a server 682, a printer 683, and the like via the LAN interface 618 and the local area network or the large area network N 1.

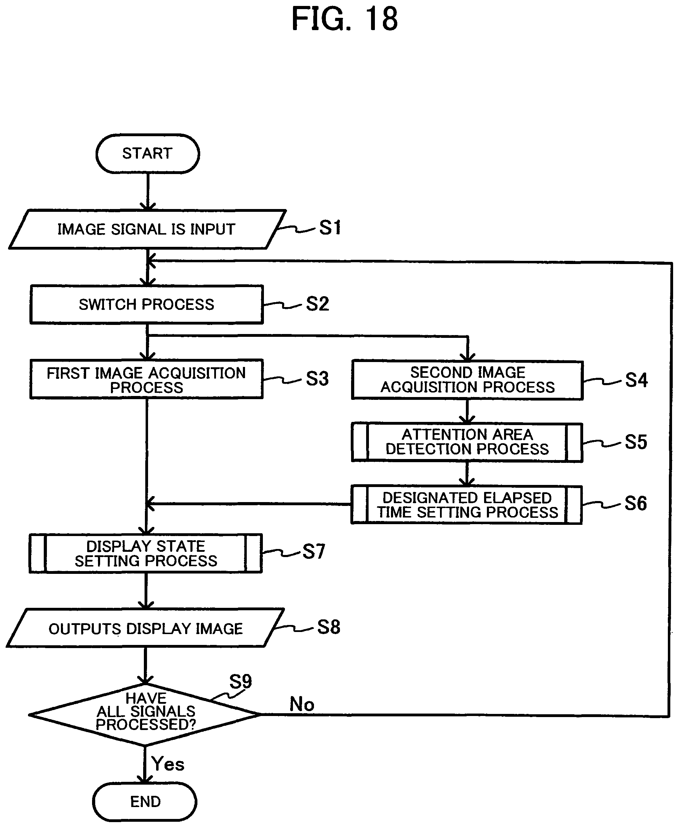

The computer system 600 implements the functions of the image processing device by reading an image processing program (e.g., an image processing program that implements a process described below with reference to FIGS. 18 to 21) recorded in a given recording medium, and executing the image processing program. The given recording medium may be an arbitrary recording medium (storage medium) that records an image processing program that can be read by the computer system 600, such as the CD-ROM 660, the USB memory 670, a portable physical medium (e.g., MO disk, DVD disk, flexible disk (FD), magnetooptical disk, or IC card), a stationary physical medium (e.g., HDD 614, RAM 612, or ROM 613) that is provided inside or outside the computer system 600, or a communication medium that temporarily stores a program during transmission (e.g., the public line N3 connected via the modem 650, or the local area network or the wide area network N1 to which the computer system (PC) 681 or the server 682 is connected).

Specifically, the image processing program is recorded in a recording medium (e.g., portable physical medium, stationary physical medium, or communication medium) so that the image processing program can be read by a computer. The computer system 600 implements the functions of the image processing device by reading the image processing program from such a recording medium, and executing the image processing program. Note that the image processing program need not necessarily be executed by the computer system 600. The invention may be similarly applied to the case where the computer system (PC) 681 or the server 682 executes the image processing program, or the computer system (PC) 681 and the server 682 execute the image processing program in cooperation.

A process performed when implementing the process of the image processing device 90 by software using an image acquired in advance is described below using the flowcharts illustrated in FIGS. 18 to 20 as an example of implementing part of the process of each section by software.

FIG. 18 is a flowchart illustrating an overall process according to one embodiment of the invention. The image signal is read (input) in a step S1. In one embodiment of the invention, an image signal output from a Bayer single-chip CCD is processed, and twenty-nine normal light images (first images) (corresponding to 29 frames) and one special light image (second image) (corresponding to one frame) are sequentially input every cycle period (=1 second) (see FIG. 8).

The normal light image/special light image switch process is then performed (step S2). A first image acquisition process (step S3) is performed when the normal light image has been input, and a second image acquisition process (step S4) is performed when the special light image has been input. In the first image acquisition process (step S3), an interpolation process, a grayscale process, and the like are performed on the normal light image (first image).

In the second image acquisition process (step S4), an interpolation process, a grayscale process, and the like are performed on the special light image (second image), and a pseudo-color image is generated. The attention area detection process is then performed (step S5). The designated elapsed time in which the alert information (alert area) that indicates information about the detection result is set to (superimposed on) the normal light image is then set (determined) (step S6).

In the display state setting process (step S7), the alert information is set to the normal light image when the current time point is within the designated elapsed time, and the alert information is not set when the current time point is not within the designated elapsed time. The display image thus generated is output (step S8). Whether or not all of the image signals have been processed is then determined. The process is performed again from the step S2 when all of the image signals have not been processed. The process ends when all of the image signals have been processed.

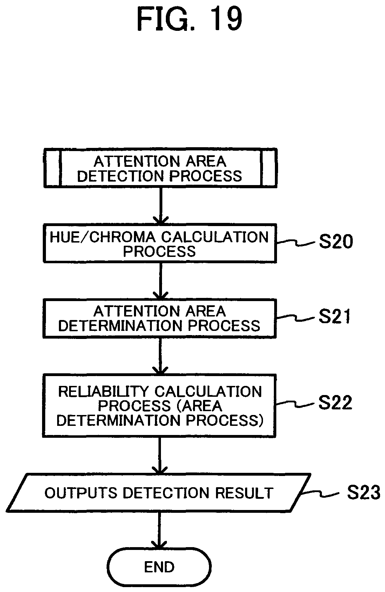

FIG. 19 is a flowchart illustrating the attention area detection process (step S5 in FIG. 18). The hue/chroma calculation process (see the expressions (1) to (5)) is performed (step S20). The threshold value determination process (see the expressions (6) and (7)) is performed to determine whether or not each pixel belongs to the attention area (step S21). The total number of pixels that belong to the attention area is then calculated. It is determined that an attention area has been detected when the total number of pixels exceeds a given threshold value, and it is determined that an attention area has not been detected when the total number of pixels is equal to or less than the given threshold value (step S22). The detection information that indicates the detection result is then output (step S23).

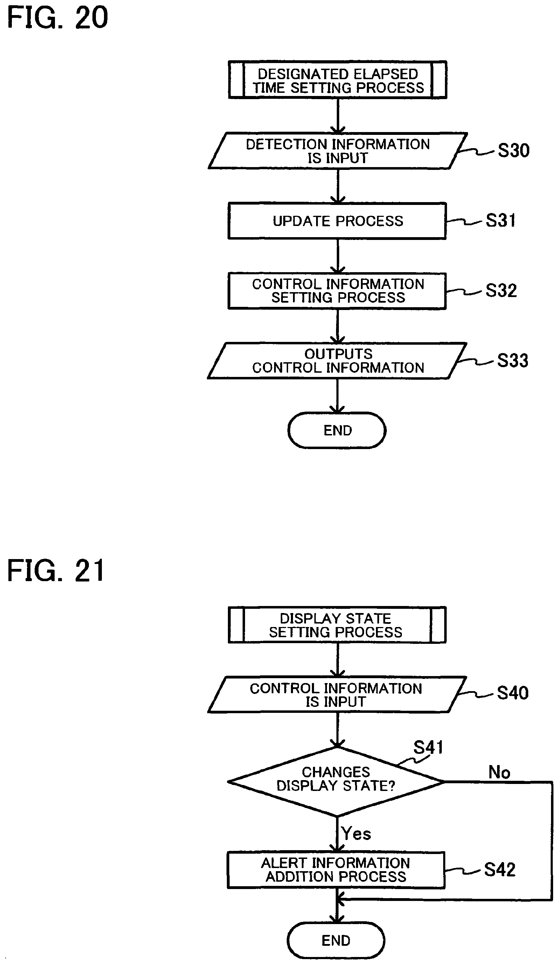

FIG. 20 is a flowchart illustrating the designated elapsed time setting process (step S6 in FIG. 18). The detection information that indicates the detection result is read in a step S30. The update process that updates the detection information (see FIGS. 13A and 13B) is then performed (step S31). Specifically, the value VD of the detection information is set to the first value VD1 (=5) when the attention area has been detected, and is decremented by one when the attention area has not been detected. When the value VD of the detection information has become a negative number, the value VD of the detection information is set to 0. The control information (about the processing method) setting process is then performed (step S32). Specifically, the control information that instructs to add the alert information is set when the value VD of the detection information is equal to or larger than 1, and the control information that instructs not to add the alert information is set when the value VD of the detection information is 0. The control information thus set is then output (step S33).

FIG. 21 is a flowchart illustrating the display state setting process (step S7 in FIG. 18). The control information about the processing method is read in a step S40. Whether or not to change the display state is determined based on the read information (step S41). When it has been determined to change the display state, the alert information addition process that superimposes an alert color (e.g., red) on the peripheral area of the normal light image is performed, and the normal light image to which the alert information is added is output as the display image (step S42). When it has been determined not to change the display state, the normal light image is directly output as the display image.

According to one embodiment of the invention, the image processing device 90 includes the first image acquisition section 110, the second image acquisition section 111, the attention area detection section 112, the designated elapsed time setting section 113, and the display state setting section 114.

The first image acquisition section 110 acquires the first image (normal light image in a narrow sense), the first image being an image that has information (signal) within the wavelength band of white light. The second image acquisition section 111 acquires the second image (special light image in a narrow sense), the second image being an image that has information (signal) within a specific wavelength band (the wavelength band of narrow-band light, fluorescence, or the like in a narrow sense). The attention area detection section 112 detects an attention area within the second image based on the feature quantity (hue, chroma, luminance, or the like in a narrow sense) of each pixel within the second image. The display state setting section 114 performs the display state setting process that sets (determines or changes) the display state of the display image (i.e., an image displayed on the display section 115) generated based on the first image, and the designated elapsed time setting section 113 performs the designated elapsed time setting process that sets (determines or changes) the designated elapsed time based on the attention area detection result (detection information) of the attention area detection section 112.

The display state setting section 114 performs the display state setting process based on the designated elapsed time that has been set by the designated elapsed time setting section 113. For example, the display state setting section 114 performs the display state setting process that changes the display state of the display image until the designated elapsed time elapses (see FIGS. 3 and 4). More specifically, the display state setting section 114 performs the display state setting process that displays the display image on the display section 115 until the designated elapsed time elapses, the alert information about the attention area being set to the display image (see FIG. 15). The term "alert information" used herein refers to information that indicates the attention area detection result. For example, the alert information (alert image) is set to the alert area that is set to the peripheral area or the like of the first image.

According to the above configuration, the user can determine that the attention area is present near the current observation position even when the attention area is detected only for a short time during observation in a moving state. This makes it possible to prevent a situation in which the attention area is missed, and reliably specify the attention area. Since the alert information is set to the peripheral area or the like of the first image, it is possible to prevent a situation in which observation of the first image is hindered, for example.

It suffices that the display image be generated using at least the first image. The display image may be an image obtained by blending the first image and the second image, for example. The display state of the display image may be changed by a method other that the alert information addition method (see FIG. 15). For example, the alert area may be set to an area of the first image other than the peripheral area, and the alert information (alert image) may be set to the alert area. A process that changes an image of a corresponding attention area within the first image may be performed as the display state change process, the corresponding attention area corresponding to the attention area detected within the second image. For example, the color of the corresponding attention area within the first image may be brought close to a given color, or a highlight process may be performed on the image of the corresponding attention area, or the first image and the second image may be blended within the corresponding attention area.

The term "attention area" used herein refers to an area for which the observation priority for the user is relatively higher than that of other areas. For example, when the user is a doctor, and desires to perform treatment, the attention area refers to an area that includes a mucosal area or a lesion area. If the doctor desires to observe bubbles or feces, the attention area refers to an area that includes a bubble area or a feces area. Specifically, the attention area for the user differs depending on the objective of observation, but necessarily has an observation priority relatively higher than that of other areas. The attention area can be detected using the feature quantity (e.g., hue or chroma) of each pixel of the second image (see the expressions (1) to (7)). For example, the threshold value of the feature quantity (see the expressions (6) and (7)) differs depending on the type of attention area. For example, the threshold value of the feature quantity (e.g., hue or chroma) of a first-type attention area differs from the threshold value of the feature quantity of a second-type attention area. When the type of attention area has changed, it suffices to change the threshold value of the feature quantity, and the process (e.g., designated elapsed time setting process and display state setting process) performed after detection of the attention area can be implemented by a process similar to the process described above.

The display state setting section 114 may perform the display state setting process that changes the display state of the display image at least until the designated elapsed time elapses even when the attention area has not been detected within the second image within the designated elapsed time (see A5 and A6 in FIG. 3).

According to the above configuration, since the display image for which the display state is changed is displayed for a while even after the attention area has become undetectable, it is possible to more effectively prevent a situation in which the user misses the attention area.

The designated elapsed time setting section 113 may set a new designated elapsed time starting from the detection timing of the attention area when the attention area has been detected within the second image within the designated elapsed time (see B3 and B4 in FIG. 4).

According to the above configuration, since the designated elapsed time is reset (extended) each time the attention area is detected, it is possible to implement an appropriate designated elapsed time setting process corresponding to detection of the attention area. Note that the new designated elapsed time may be set at a timing within the cycle period subsequent to the cycle period in which the attention area has been detected, or may be set at a timing within the cycle period in which the attention area has been detected.

The first image acquisition section 110 may acquire the first image corresponding to at least one frame (i.e., at least one first image) every cycle period (image acquisition period), and the second image acquisition section 111 may acquire the second image corresponding to at least one frame (i.e., at least one second image) every cycle period (see FIG. 8). The display state setting section 114 may perform the display state setting process that changes the display state of the display image in an (N+1)th (N is a natural number) cycle period (TN+1) subsequent to an Nth cycle period (TN) when the attention area has been detected within the second image in the Nth cycle period.

It is possible to notify the user of detection of the attention area by thus performing the attention area detection process in each cycle period, and changing the display state of the display image in the next and subsequent cycle periods when the attention area has been detected. This makes it possible to minimize a delay time when displaying the alert information about the attention area or the like to the user, and more effectively prevent a situation in which the user misses the attention area.

The first image acquisition section 110 may acquire the first images corresponding to K frames (K is a natural number) every cycle period, and the second image acquisition section 111 may acquire the second images corresponding to L frames (L is a natural number) every cycle period. For example, the second image acquisition section 111 acquires the second image corresponding to one frame (L=1) every cycle period. The relationship "K>L" may be satisfied (see FIG. 8).

It is possible to acquire the normal light image in a high ratio as compared with the special light image by thus acquiring the first images corresponding to K frames and the second images corresponding to L frames every cycle period so that the relationship "K>L" is satisfied. This makes it possible to prevent a decrease in temporal resolution of the normal light image, and obtain a high-quality display image (e.g., moving image).

The relationship "TE>T" may be satisfied when the length of each cycle period is referred to as T, and the length of the designated elapsed time is referred to as TE (see FIG. 3).

In this case, since the designated elapsed time can be made longer than the cycle period (i.e., attention area detection unit period), a period in which the display state is changed increases. This makes it possible to effectively prevent a situation in which the user misses the attention area.

The display state setting section 114 may perform the display state setting process that changes the display state of the display image in (N+1)th to Mth cycle periods (TN+1 to TN+5; M is an integer that satisfies "M>N+1") when the attention area has been detected within the second image in the Nth cycle period (TN), and the attention area has not been detected within the second image in the (N+1)th cycle period (TN+1) (see C1, C2, and C3 in FIG. 9A).

In this case, when the attention area has been detected in the (N+1)th cycle period (TN+1), the display state of the display image is changed at least until the Mth cycle period (TN+5) elapses (i.e., the designated elapsed time can be made longer than the cycle period).

The designated elapsed time setting section 113 may include the detection information recording section 301 that records the detection information about the attention area, the update section 300 that performs the update process that updates the detection information based on the detection result output from the attention area detection section 112, and the control information output section 302 that outputs the control information that controls the display state setting process performed by the display state setting section 114 based on the detection information recorded in the detection information recording section 301 (see FIG. 12).

This makes it possible to implement the designated elapsed time setting process based on the detection information about the attention area. Since the detection information about the attention area is recorded (stored) in the detection information recording section 301 when the attention area has been detected, it is possible to set the designated elapsed time by utilizing the detection information stored in the detection information recording section 301 even when the attention area has become undetectable.

The update section 300 may set the value VD of the detection information to the first value VD1 (e.g., VD1=5) that corresponds to the designated elapsed time (see FIG. 13) when the attention area has been detected within the second image. The update section 300 may perform the update process that sequentially changes (e.g., decrements or increments) the value VD of the detection information from the first value VD1 to the second value VD2 (e.g., VD2=0) when the attention area has not been detected within the second image. For example, the update section 300 performs the update process that updates the detection information every cycle period. More specifically, the update section 300 sets the value VD to the first value VD1 when the attention area has been detected in the Nth cycle period (TN), and performs the update process that sequentially changes the value VD from the first value VD1 to the second value VD2 when the attention area has not been detected in the cycle period (TN+1, TN+2, ...) subsequent to the Nth cycle period (TN).

The control information output section 302 may output the control information (control signal or control flag) that instructs the display state setting section 114 to change the display state of the display image until the value VD of the detection information reaches the second value VD2, and may output the control information that instructs the display state setting section 114 not to change the display state of the display image when the value VD of the detection information has reached the second value VD2.

It is possible to efficiently implement the designated elapsed time setting process (see FIGS. 3 and 4, for example) by thus updating the value of the detection information recorded in the detection information recording section 301.

The update section 300 may reset the value VD of the detection information to the first value VD1 (e.g., VD=VD1=5) when the attention area has been detected within the second image in the cycle period (TN+1, TN+2, ...) subsequent to the Nth cycle period (TN) (see FIG. 13B).

This makes it possible to implement the process that sets the designated elapsed time when the attention area has been detected within the designated elapsed time by resetting the value of the detection information. The designated elapsed time can be extended by resetting the value of the detection information until the attention area becomes undetectable.

The display state setting section 114 may include the processing section 400 (see FIG. 14). The processing section 400 may process the first image (e.g., add the alert information to the first image), and output the processed first image as the display image when the control information output from the control information output section 302 (see FIG. 12) instructs to change the display state of the display image. The processing section 400 may directly output the first image as the display image when the control information instructs not to change the display state of the display image.

It is possible to implement the display state change process based on detection of the attention area and the designated elapsed time by thus causing the control information output section 302 included in the designated elapsed time setting section 113 to output the control information, and causing the processing section 400 included in the display state setting section 114 to process the first image based on the control information. Since it suffices that the processing section 400 process the first image in accordance with the control information output from the designated elapsed time setting section 113, the configuration and the process of the processing section 400 can be simplified.

The attention area detection section 112 may include the reliability calculation section 205 (see FIG. 10). The reliability calculation section 205 may calculate the reliability of the attention area detected by the attention area detection section 112 (i.e., an index (measure) that indicates the reliability of the attention area). More specifically, the reliability calculation section 205 calculates the reliability based on the area of the attention area detected by the attention area detection section 112.

This makes it possible to improve the accuracy of the attention area (e.g., accuracy when determining a lesion area as an attention area) as compared with a method that does not utilize the reliability. Since a large area is detected as the attention area, and a small area is not detected as the attention area, the effects of noise can be reduced, for example.

Although an example in which the designated elapsed time is set to 5 cycle periods (5 seconds) when an attention area has been detected has been described above, the designated elapsed time may be set to an arbitrary value. The user may set an arbitrary designated elapsed time via the external I/F section 117. Although an example in which the rotary filter 103 rotates one revolution per second, and the area ratio of the normal light image filter F1 to the special light image filter F2 in the circumferential direction is 29:1 has been described above, the rotation speed of the rotary filter 103 and the area ratio of the normal light image filter F1 to the special light image filter F2 may be set arbitrarily. For example, the ratio of the special light image (i.e., the ratio of L to K) may be increased while giving priority to the temporal resolution of the normal light image.

Although an example in which a single-chip CCD in which a Bayer primary color filter is disposed on the front side is used as the imaging system has been described above, the imaging system is not limited thereto. For example, a double-chip or triple-chip CCD may also be used. Although an example in which the special light image is acquired using the narrow band of blue light and the narrow band of green light as disclosed in JP-A-2002-95635 has been described above, another configuration may also be employed. For example, the special light image may be acquired using fluorescence (see JP-A-63-122421), infrared light, or the like. Although an example in which the rotary filter 103 is used to capture the normal light image and the special light image has been described above, another configuration may also be employed. For example, white light and narrow-band light may be applied by changing the light source (e.g., LED light source).

Although an example in which the image processing device is integrated with the imaging section (lens system 100, CCD 101, illumination light source 102, rotary filter 103, gain amplifier 104, A/D conversion section 105, WB section 107, and photometrical evaluation section 108) has been described above, another configuration may also be employed. For example, an image signal acquired using a separate imaging section (e.g., capsule endoscope) may be stored in a recording medium in raw data format, and the image signal read from the recording medium may be processed.

The above embodiments may also be applied to a program that causes a computer to function as each section (e.g., first image acquisition section, second image acquisition section, attention area detection section, display state setting section, designated elapsed time setting section, and moving amount detection section) described above.

In this case, it is possible to store image data in advance (e.g., capsule endoscope), and process the stored image data by software using a computer system (e.g., PC).

The above embodiments may also be applied to a computer program product that stores a program code that implements each section (e.g., first image acquisition section, second image acquisition section, attention area detection section, display state setting section, designated elapsed time setting section, and moving amount detection section) described above.

The term "computer program product" used herein refers to an information storage medium, a device, an instrument, a system, or the like that stores a program code, such as an information storage medium (e.g., optical disk medium (e.g., DVD), hard disk medium, and memory medium) that stores a program code, a computer that stores a program code, or an Internet system (e.g., a system including a server and a client terminal), for example. In this case, each element and each process according to the above embodiments are implemented by respective modules, and a program code that includes these modules is recorded in the computer program product.

3. Second configuration example

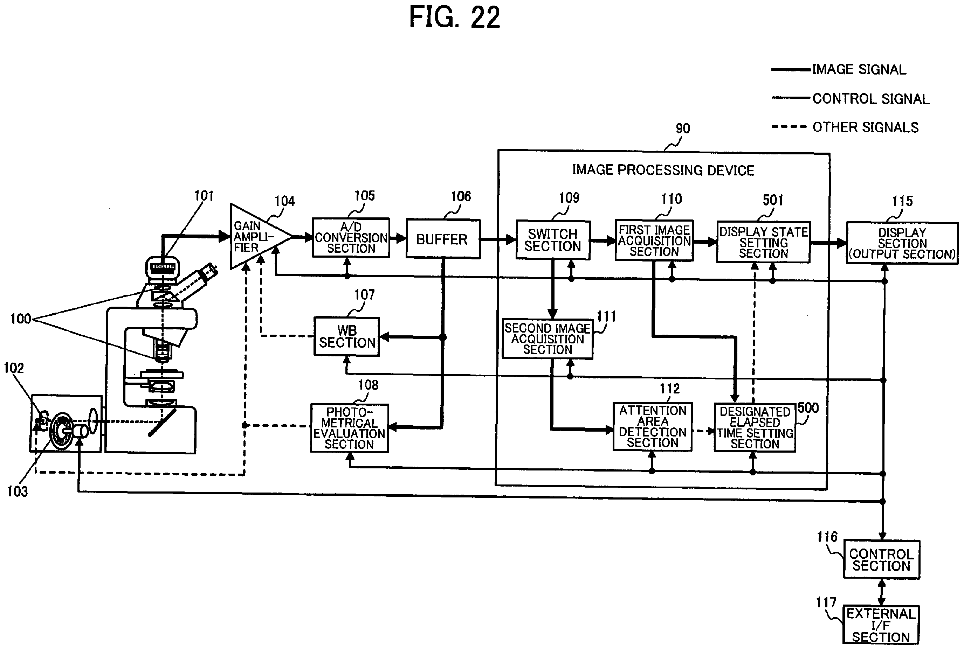

FIG. 22 illustrates a second configuration example according to one embodiment of the invention. In the second configuration example, the image processing device 90 according to one embodiment of the invention is applied to a microscope system. Note that the second configuration example may also be applied to another electronic system such as an endoscope system (see FIG. 5).

The second configuration example illustrated in FIG. 22 differs from the first configuration example illustrated in FIG. 5 in that the designated elapsed time setting section 113 and the display state setting section 114 are replaced with a designated elapsed time setting section 500 and a display state setting section 501. The basic configuration of the second configuration example is the same as that of the first configuration example, and an element identical with that of the first configuration example is indicated by an identical name and an identical reference symbol. The differences from the first configuration example are mainly described below.

An image signal obtained via the lens system 100 and the CCD 101 of the microscope is amplified by the gain amplifier 104, and converted into a digital signal by the A/D conversion section 105. Illumination light emitted from the illumination light source 102 passes through the filter attached to the rotary filter 103, and is guided to an objective stage of the microscope. The first image acquisition section 110 is connected to the designated elapsed time setting section 500 and the display state setting section 501. The display state setting section 501 is connected to the display section 115. The attention area detection section 112 is connected to the designated elapsed time setting section 500, and the designated elapsed time setting section 500 is connected to the display state setting section 501. The control section 116 is bidirectionally connected to the designated elapsed time setting section 500 and the display state setting section 501.

An operation according to the second configuration example is described below. Note that the operation according to the second configuration example is basically the same as the operation according to the first configuration example. The differences from the first configuration example are mainly described below.

The first image acquisition section 110 reads the normal light image from the switch section 109, performs an interpolation process, a grayscale process, and the like on the normal light image, and transmits the resulting normal light image to the designated elapsed time setting section 500 and the display state setting section 501 under control of the control section 116. The attention area detection section 112 reads the special light image from the second image acquisition section 111, and performs the attention area detection process that detects an attention area (e.g., a lesion area in which blood vessels are densely present) under control of the control section 116. The attention area detection result is transmitted to the designated elapsed time setting section 500.

The designated elapsed time setting section 500 reads the attention area detection result from the attention area detection section 112, and reads two normal light images from the first image acquisition section 110 under control of the control section 116. In one embodiment of the invention, twenty-nine normal light images and one special light image are obtained in one cycle period (1 second) (see FIG. 8). Therefore, twenty-nine normal light images correspond to one special light image. The first normal light image and the second normal light corresponding to two frames are read in order to calculate the motion amount of the normal light image in one cycle period.

The designated elapsed time setting section 500 determines the designated elapsed time while taking account of the motion amount calculated from the two normal light images in addition to the detection result read from the attention area detection section 112. More specifically, the designated elapsed time setting section 500 determines whether or not the current time point is within the set designated elapsed time, and transmits the determination result to the display state setting section 501. The display state setting section 501 reads the determination result as to whether or not the current time point is within the designated elapsed time from the designated elapsed time setting section 113, and selects the alert information addition process when the current time point is within the designated elapsed time under control of the control section 116. An edge/chroma enhancement process is performed on the normal light image as the alert information addition process (alert area superimposition process). The display state setting section 501 does not perform a process when the current time point is not within the designated elapsed time. The display image output from the display state setting section 501 is transmitted to the display section 115, and sequentially displayed on the display section 115.

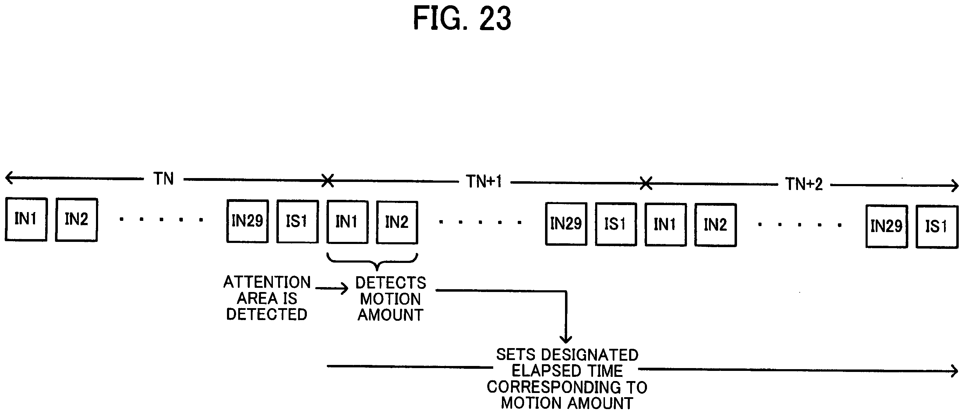

FIG. 23 is a view illustrating a designated elapsed time setting method according to the second configuration example. In the second configuration example, the motion amount of the normal light image is detected, and the designated elapsed time setting process is performed based on the detected motion amount. More specifically, the designated elapsed time setting section 113 performs the designated elapsed time setting process that reduces the designated elapsed time as the motion amount increases. The motion amount of the normal light image is detected on condition that an attention area has been detected within the special light image.

In FIG. 23, an attention area has been detected within the special light image IS1 acquired in the cycle period TN. In this case, the motion amount is detected using the normal light images IN1 and IN2 acquired in the next cycle period TN+1, for example. The designated elapsed time corresponding to the detected motion amount is set, and the display state change process is performed within the designated elapsed time in the same manner as in the first configuration example.

When the designated elapsed time is set based on the motion amount of the normal light image, the designated elapsed time decreases (i.e., a period of time in which the display state is changed decreases) when the imaging section (camera gaze point) moves at high speed with respect to the object. Therefore, since a period of time in which the display state is changed decreases when the imaging section moves at high speed with respect to the object, and the attention area is not likely to be present near the current observation position, convenience to the user can be improved.

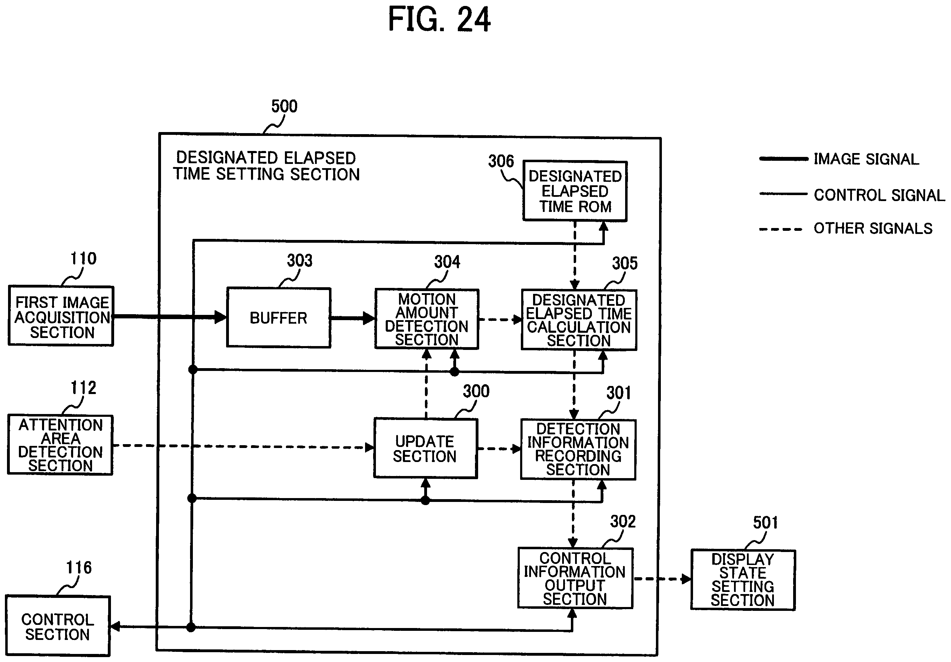

FIG. 24 illustrates a configuration example of the designated elapsed time setting section 500. Note that various modifications may be made, such as omitting some of the elements illustrated in FIG. 24 or adding other elements.

The designated elapsed time setting section 500 illustrated in FIG. 24 differs from the designated elapsed time setting section 113 illustrated in FIG. 12 in that a buffer 303, a motion amount detection section 304, a designated elapsed time calculation section 305, and a designated elapsed time ROM 306 are additionally provided. The basic configuration of the designated elapsed time setting section 500 illustrated in FIG. 24 is the same as that of the designated elapsed time setting section 113 illustrated in FIG. 12, and an element identical with that of the designated elapsed time setting section 113 is indicated by an identical name and an identical reference symbol. The differences from the designated elapsed time setting section 113 are mainly described below.

The first image acquisition section 110 is connected to the motion amount detection section 304 via the buffer 303. The motion amount detection section 304 and the designated elapsed time ROM 306 are connected to the designated elapsed time calculation section 305. The designated elapsed time calculation section 305 is connected to the detection information recording section 301. The update section 300 is connected to the detection information recording section 301 and the motion amount detection section 304. The control information output section 302 is connected to the display state setting section 501. The control section 116 is bidirectionally connected to the motion amount detection section 304, the designated elapsed time calculation section 305, and the designated elapsed time ROM 306.

When the image processing device has been initialized upon power on, for example, the control section 116 initializes the detection information recording section 301 to set the value of the detection information to an initial value "0". The update section 300 reads the detection information that indicates whether or not an attention area has been detected within the special light image from the attention area detection section 112 under control of the control section 116.

The update section 300 transmits a control signal that instructs to calculate the motion amount to the motion amount detection section 304 when an attention area has been detected. The update section 300 decrements the value of the detection information recorded in the detection information recording section 301 by one when an attention area has not been detected. When the value of the detection information has become a negative number, the value of the detection information is set to 0.

The motion amount detection section 304 calculates the motion amount of the normal light image stored in the buffer 303 only when the control signal has been transmitted from the update section 300. The buffer 303 stores the first normal light image IN1 and the second normal light image IN2 in each cycle period (see FIG. 23). Note that the motion amount is calculated using a known block matching technique or the like. The motion amount thus calculated is transmitted to the designated elapsed time calculation section 305. The designated elapsed time calculation section 305 reads a relationship table that links the motion amount and the designated elapsed time from the designated elapsed time ROM 306 under control of the control section 116.

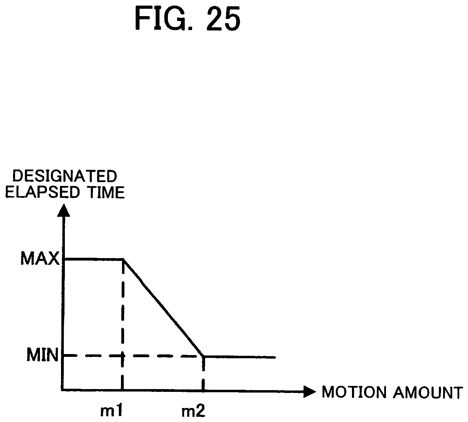

FIG. 25 illustrates an example of the relationship table that links the motion amount and the designated elapsed time (cycle period). As illustrated in FIG 25, the designated elapsed time normally decreases as the motion amount increases. Note that the upper-limit value (MAX) and the lower-limit value (MIN) are set to the designated elapsed time. The designated elapsed time is set to the upper-limit value (MAX) when the motion amount is equal to or smaller than a given value (m1), and is set to the lower-limit value (MIN) when the motion amount is equal to or larger than a given value (m2). The designated elapsed time in which the display state is changed decreases as the motion amount increases by utilizing such a relationship table. Since the distance from the detected attention area increases as the moving speed increases, it is necessary to prevent a situation in which the possibility that the attention area is present near the current observation position decreases to a large extent.

The designated elapsed time calculation section 305 calculates the designated elapsed time with respect to the motion amount calculated by the motion amount detection section 304 based on the relationship table read from the designated elapsed time ROM 306, and outputs the calculated designated elapsed time to the detection information recording section 301 as the value of the detection information. In FIG. 13 A, the first value VD1 of the detection information is fixed at 5 (D1). In the second configuration example, the first value VD1 is variably set. More specifically, the first value VD1 and the designated elapsed time that is set using the first value VD1 decrease as the motion amount of the normal light image increases.

The control information output section 302 reads the value of the detection information from the detection information recording section 301 under control of the control section 116. The control information output section 302 outputs the control information that instructs the display state setting section 501 to change the display state to the display state setting section 501 when the value of the detection information is equal to or larger than 1. The control information output section 302 outputs the control information that instructs the display state setting section 501 not to change the display state to the display state setting section 501 when the value of the detection information is 0.

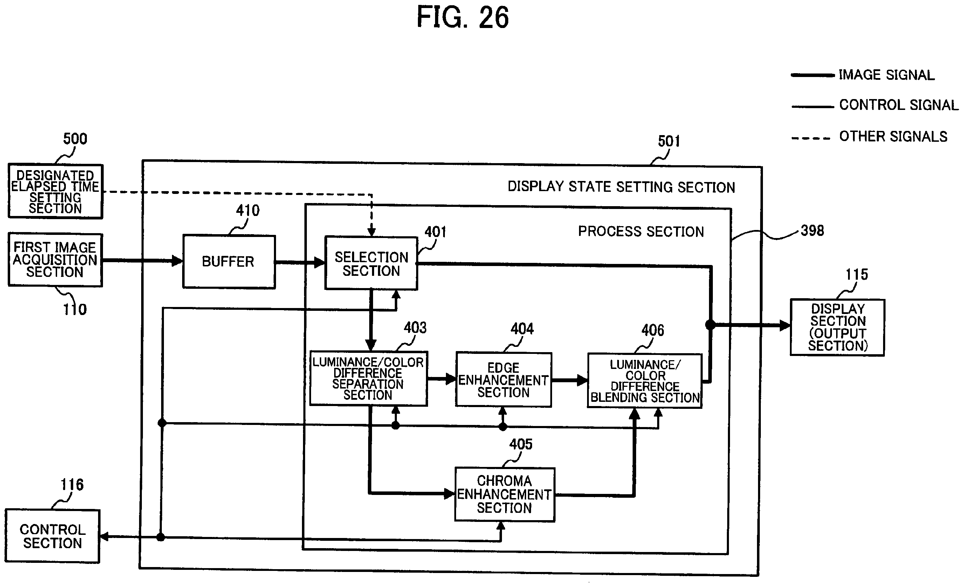

FIG. 26 illustrates a configuration example of the display state setting section 501. A processing section 398 illustrated in FIG. 26 differs in configuration from the processing section 400 illustrated in FIG. 14. The processing section 398 illustrated in FIG. 26 includes a luminance/color difference separation section 403, an edge enhancement section 404, a chroma enhancement section 405, and a luminance/color difference blending section 406, but does not include the alert information addition section 402. The basic configuration of the processing section 398 illustrated in FIG. 26 is the same as that of the processing section 400 illustrated in FIG. 14, and an element identical with that of the processing section 400 illustrated in FIG. 14 is indicated by an identical name and an identical reference symbol. The differences from the processing section 400 are mainly described below.

The designated elapsed time setting section 500 is connected to the selection section 401. The selection section 401 is connected to the luminance/color difference separation section 403 and the display section 115. The luminance/color difference separation section 403 is connected to the edge enhancement section 404 and the chroma enhancement section 405. The edge enhancement section 404 and the chroma enhancement section 405 are connected to the luminance/color difference blending section 406. The luminance/color difference blending section 406 is connected to the display section 115. The control section 116 is bidirectionally connected to the luminance/color difference separation section 403, the edge enhancement section 404, the chroma enhancement section 405, and the luminance/color difference blending section 406.

The selection section 401 reads the control information that instructs whether or not to change the display state from the designated elapsed time setting section 500, and reads the normal light image from the buffer 410 under control of the control section 116. When the control information that instructs to change the display state has been read from the designated elapsed time setting section 500, the selection section 401 transmits the normal light image to the luminance/color difference separation section 403. When the control information that instructs not to change the display state has been read from the designated elapsed time setting section 500, the selection section 401 transmits the normal light image to the display section 115.

The luminance/color difference separation section 403 converts the R signal, the G signal, and the B signal of the normal light image into the luminance signal Y and the color difference signals Cb and Cr (see the expressions (1) to (3)) under control of the control section 116 when the normal light image has been transmitted from the selection section 401. The luminance signal Y is transmitted to the edge enhancement section 404, and the color difference signals Cb and Cr are transmitted to the chroma enhancement section 405.

The edge enhancement section 404 performs a known edge enhancement process on the luminance signal Y under control of the control section 116. A luminance signal Y' obtained by the edge enhancement process is transmitted to the luminance/color difference blending section 406. The chroma enhancement section 405 performs a known chroma enhancement process on the color difference signals Cb and Cr under control of the control section 116. Color difference signals Cb' and Cr' obtained by the chroma enhancement process are transmitted to the luminance/color difference blending section 406.

The luminance/color difference blending section 406 blends the luminance signal Y' from the edge enhancement section 404 and the color difference signals Cb' and Cr' from the chroma enhancement section 405 to generate an R' signal, a G' signal, and a B' signal (see the expressions (7) to (9)), and outputs the resulting signal as the display image under control of the control section 116.

$$R' = Y' + 1.40200Cr' \qquad (7)$$

$$G' = Y' - 0.34414Cb' - 0.71414Cr' \qquad (8)$$

$$B' = Y' + 1.77200Cb' \qquad (9)$$

In the second configuration example, the alert information is thus set to the entire normal light image.

According to the second configuration example, the designated elapsed time setting section 500 includes the motion amount detection section 304. The motion amount detection section 304 detects the motion amount of the first image acquired by the first image acquisition section 110.

The designated elapsed time setting section 500 performs the designated elapsed time setting process based on the motion amount detected by the motion amount detection section 304 (see FIG. 23). More specifically, the designated elapsed time setting section 500 performs the designated elapsed time setting process that reduces the designated elapsed time as the motion amount increases.

According to the above configuration, since the designated elapsed time is set based on the motion amount of the first image, the designated elapsed time (i.e., a period of time in which the display state is changed) is reduced when the motion amount of the first image is large (i.e., when it is determined that the imaging section moves at high speed). This makes it possible to provide a convenient image processing device that can prevent a situation in which the alert information is frequently displayed when the imaging section moves at high speed.

The motion amount detection section 304 may detect the motion amount of the first image when an attention area has been detected within the second image.

According to the above configuration, the motion amount of the first image is not detected when an attention area has been detected. This makes it possible to prevent a situation in which the heavy-load motion amount detection process is unnecessarily performed. Therefore, an intelligent designated elapsed time setting process using the motion amount of the first image can be implemented with reduced processing load.

The designated elapsed time setting section 500 may include the detection information recording section 301, the update section 300, and the control information output section 302 in addition to the motion amount detection section 304 (see FIG 24). The update section 300 may set the value of the detection information based on the motion amount detected by the motion amount detection section 304.

More specifically, the update section 300 may set the value VD of the detection information to the first value VD1 that changes depending on the detected motion amount when the attention area has been detected within the second image. Specifically, the update section 300 sets the value VD of the detection information to the first value VD1 when an attention area has been detected (see D1 in FIG. 13A). In the second configuration example, the first value VD1 changes depending on the motion amount (e.g., the first value VD1 decreases as the motion amount increases). The update section 300 performs the update process that sequentially changes the value VD of the detection information from the first value VD1 to the second value VD2 when an attention area has not been detected within the second image (see D6 and D7 in FIG. 13A).

The control information output section 302 may output the control information that instructs to change the display state of the display image until the value VD of the detection information reaches the second value VD2 (=0), and may output the control information that instructs not to change the display state of the display image when the value VD of the detection information has reached the second value VD2.

It is possible to efficiently implement the designated elapsed time setting process corresponding to the motion amount by thus updating the value of the detection information recorded in the detection information recording section 301. Since the designated elapsed time setting process corresponding to the motion amount can be implemented by merely setting the first value VD1 that changes corresponding to the motion amount to the detection information recording section 301, the process and the configuration can be simplified.

The display state setting section 501 illustrated in FIG. 26 may enhance at least one of the edge and the chroma of the first image.

It is possible to provide an image processing device that is convenient to the user by thus performing the display state change process that enhances the edge/chroma of the entire normal light image to generate the display image.

Although an example in which the designated elapsed time is set using the motion amount of the normal light image has been described above, another configuration may also be employed. For example, the designated elapsed time may be fixed in the same manner as in the first configuration example. The motion amount of the normal light image may also be used in the first configuration example.

Although an example in which the display state change process enhances the edge/chroma of the entire normal light image has been described above, another configuration may also be employed. For example, the alert area may be added to the peripheral area of the normal light image in the same manner as in the first configuration example. The edge/chroma of the entire normal light image may also be enhanced in the first configuration example. Although an example in which the process is implemented by hardware has been described above, another configuration may also be employed. For example, the process may be implemented by software as described above in connection with the first configuration example.

4. Third configuration example

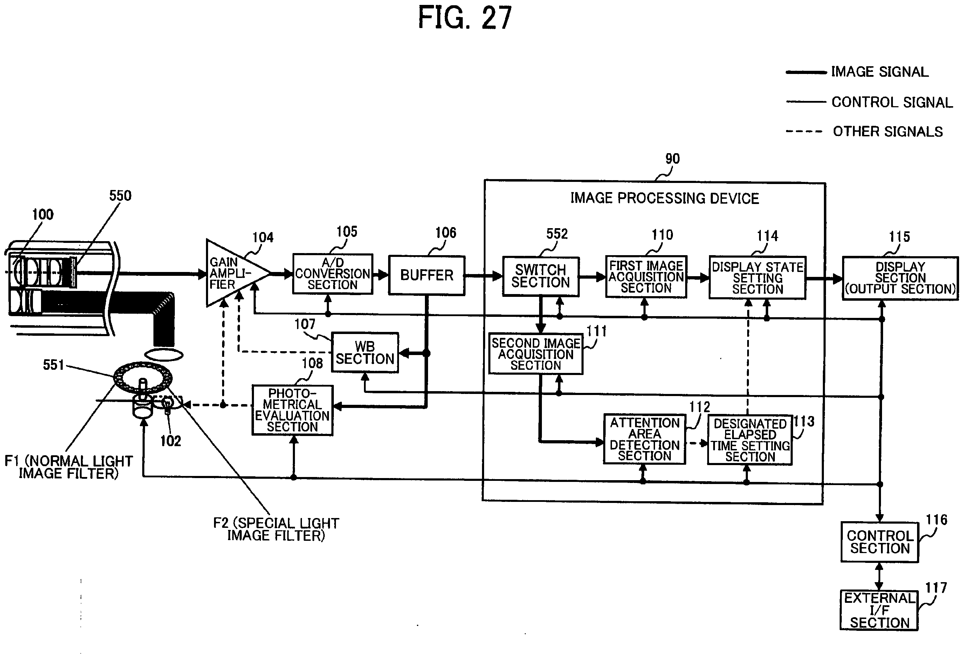

FIG. 27 illustrates a third configuration example according to one embodiment of the invention. FIG. 27 illustrates an example in which one embodiment of the invention is applied to a frame-sequential endoscope system. The third configuration example differs from the first configuration example illustrated in FIG 5 in that the CCD 101, the rotary filter 103, and the switch section 109 are respectively replaced with a CCD 550, a rotary filter 551, and a switch section 552. The third configuration example is basically the same as the first configuration example, and an element identical with that of the first configuration example is indicated by an identical name and an identical reference symbol. The differences from the first configuration example are mainly described below.

An image signal obtained via the lens system 100 and the CCD 550 provided on the end of the endoscope is amplified by the gain amplifier 104, and converted into a digital signal by the A/D conversion section 105. Illumination light emitted from the illumination light source 102 passes through the filter attached to the rotary filter 551 provided on the end of the endoscope, and is applied to an object via an optical fiber. The control section 116 is bidirectionally connected to the rotary filter 551 and the switch section 552.

An operation according to the third configuration example is described below. Note that the operation according to the third configuration example is basically the same as the operation according to the first configuration example. The differences from the first configuration example are mainly described below.

The image signal obtained via the lens system 100 and the CCD 550 is output as an analog signal. In one embodiment of the invention, the CCD 550 is a single-chip monochromatic CCD, and the illumination light source 102 is a normal white light source (e.g., xenon lamp).

The rotary filter 551 is provided with twenty-nine sets of filters respectively having the R, G, and B spectral characteristics of the normal light image. The rotary filter 551 is also provided with one set of a filter that allows light within the narrow band (390 to 445 nm) of blue light to pass through, a filter that allows light within the narrow band (530 to 550 nm) of green light to pass through, and a light-shielding filter in the same manner as in the first configuration example.

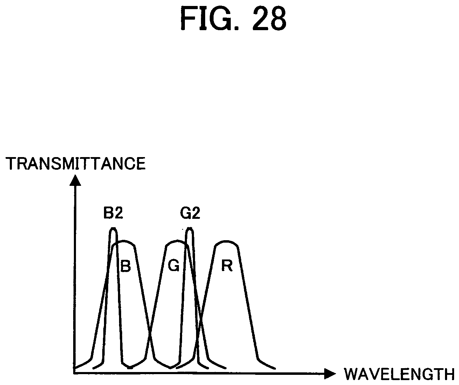

FIG. 28 illustrates the spectral characteristics of the R filter, the G filter, the B filter, the blue-light narrow-band filter (B2), and the green-light narrow-band filter (G2). The CCD 550 captures an image at intervals of 1/90th of a second, and the rotary filter 551 rotates one revolution per second.

Twenty-nine normal light images (R, G, and B signals) are acquired while the rotary filter 551 rotates one revolution (1/30th of a second (=3×1/90)). The special light image is captured using the light-shielding filter (R signal), the green-light narrow-band filter (G2) (G signal), and the blue-light narrow-band filter (B2) (B signal). Therefore, one special light image is acquired while the rotary filter 551 rotates one revolution (1/30th of a second (=3×1/90)).

The buffer 106 can store (record) one normal light image or one special light image, and a new image acquired by the imaging operation is overwritten. The switch section 552 transmits the normal light image (R, G, and B signals) stored in the buffer 106 to the first image acquisition section 110 under control of the control section 116. When the special light image that includes a blue-light narrow-band component and a green-light narrow-band component is stored in the buffer 106, the switch section 552 transmits the special light image to the second image acquisition section 111.

The first image acquisition section 110 reads the normal light image from the switch section 552, performs a grayscale process and the like on the normal light image, and transmits the resulting normal light image to the display state setting section 114 under control of the control section 116. The second image acquisition section 111 reads the special light image from the switch section 552, and performs a grayscale process and the like on the special light image under control of the control section 116. The second image acquisition section 111 also performs a process that generates a pseudo-color image. The subsequent process is performed in the same manner as in the first configuration example illustrated in FIG. 5.

According to the third configuration example, the normal light image and the special light image are acquired, and an attention area is detected based on the feature quantity of each pixel of the special light image. The designated elapsed time is determined (set) based on the detection result. The display state of the display image is set based on the determined designated elapsed time. Therefore, the user can determine that the attention area is present near the current observation position even when the attention area is detected only for a short time during observation in a moving state. This makes it possible to prevent a situation in which the attention area is missed, and reliably specify the attention area. Moreover, the normal light image and the special light image are acquired every given cycle period so that the normal light image is acquired in a high ratio as compared with the special light image. This makes it possible to prevent a decrease in temporal resolution of the normal light image, and obtain a high-quality display image. Since the alert information is set to the peripheral area of the normal light image, it is possible to provide an image processing device that ensures excellent operability, and does not hinder observation of the normal light image.

Although the first to third configuration examples according to several embodiments of the invention have been described in detail above, those skilled in the art would readily appreciate that many modifications are possible in the above embodiments.

For example, the specific wavelength band may be narrower than the wavelength band of white light (narrow band imaging (NBI)). The normal light image and the special light image may be an *in vivo* image, and the specific wavelength band included in the *in vivo* image may be the wavelength band of light absorbed by hemoglobin in blood, for example. The wavelength band of light absorbed by hemoglobin may be 390 to 445 nm (first narrow-band light or a B2 component of narrow-band light) or 530 to 550 nm (second narrow-band light or a G2 component of narrow-band light), for example.

This makes it possible to observe the structure of blood vessels positioned in a surface area and a deep area of tissue. A lesion area (e.g., epidermoid cancer) that is difficult to observe using normal light can be displayed in brown or the like by inputting the resulting signal to a given channel (G2→R, B2→G and B), so that the lesion area can be reliably detected. A wavelength band of 390 to 445 nm or 530 to 550 nm is selected from the viewpoint of absorption by hemoglobin and the ability to reach a surface area or a deep area of tissue. Note that the wavelength band is not limited thereto. For example, the lower limit of the wavelength band may decrease by about 0 to 10%, and the upper limit of the wavelength band may increase by about 0 to 10%, depending on a variation factor (e.g., experimental results for absorption by hemoglobin and the ability to reach a surface area or a deep area of tissue).

The specific wavelength band included in the *in vivo* image may be the wavelength band of fluorescence emitted from a fluorescent substance. For example, the specific wavelength band may be 490 to 625 nm.

This makes it possible to implement autofluorescence imaging (AFI). Intrinsic fluorescence (490 to 625 nm) from a fluorescent substance (e.g., collagen) can be observed by applying excitation light (390 to 470 nm). In this case, the lesion area can be highlighted in a color differing from that of a normal mucous membrane, so that the lesion area can be reliably detected, for example. A wavelength band of 490 to 625 nm is the wavelength band of fluorescence produced by a fluorescent substance (e.g., collagen) when excitation light is applied. Note that the wavelength band is not limited thereto. For example, the lower limit of the wavelength band may decrease by about 0 to 10%, and the upper limit of the wavelength band may increase by about 0 to 10% depending on a variation factor (e.g., experimental results for the wavelength band of fluorescence produced by a fluorescent substance). A pseudo-color image may be generated by simultaneously applying light within a wavelength band (540 to 560 nm) that is absorbed by hemoglobin.

The specific wavelength band included in the *in vivo* image may be the wavelength band of infrared light. For example, the specific wavelength band may be 790 to 820 nm or 905 to 970 nm.

This makes it possible to implement infrared imaging (IRI). Information about a blood vessel or a blood flow in a deep area of a mucous membrane that is difficult to observe visually, can be highlighted by intravenously injecting indocyanine green (ICG) (infrared marker) that easily absorbs infrared light, and applying infrared light within the above wavelength band, so that the depth of gastric cancer invasion or the therapeutic strategy can be determined, for example. An infrared marker exhibits maximum absorption in a wavelength band of 790 to 820 nm, and exhibits minimum absorption in a wavelength band of 905 to 970 nm. Note that the wavelength band is not limited thereto. For example, the lower limit of the wavelength band may decrease by about 0 to 10%, and the upper limit of the wavelength band may increase by about 0 to 10% depending on a variation factor (e.g., experimental results for absorption by the infrared marker).

The second image acquisition section 111 (see FIG. 5, for example) may generate the special light image (second image) based on the acquired white light image (first image).

More specifically, the second image acquisition section 111 may include a signal extraction section that extracts a signal within the wavelength band of white light from the acquired white light image, and the second image acquisition section may generate the special light image that includes a signal within the specific wavelength band based on the signal within the wavelength band of white light extracted by the signal extraction section. For example, the signal extraction section may estimate the spectral reflectance characteristics of the object from the RGB signals of the white light image at intervals of 10 nm, and the second image acquisition section 111 may integrate the estimated signal components within the specific wavelength band to generate the special light image.

The second image acquisition section 111 may include a matrix data setting section that sets matrix data for calculating a signal within the specific wavelength band from a signal within the wavelength band of white light. The second image acquisition section 111 may calculate a signal within the specific wavelength band from a signal within the wavelength band of white light using the matrix data set by the matrix data setting section to generate the special light image. For example, the matrix data setting section may set table data as the matrix data, the spectral characteristics of illumination light within the specific wavelength band being described in the table data at intervals of 10 nm. The estimated spectral reflectance characteristics of the object may be multiplied by the spectral characteristics (coefficient) described in the table data to generate the special light image.

In this case, since the special light image can be generated based on the normal light image, it is possible to implement a system using only one light source that emits normal light and one image sensor that captures normal light. This makes it possible to reduce the size of an insertion section of a scope endoscope or a capsule endoscope. Moreover, since the number of parts can be reduced, a reduction in cost can be achieved.

Although an example in which the alert information is an image (see FIG. 15) has been described above, the alert information is not limited to an image. The alert information may be alert information using sound, or alert information using light emitted from a light-emitting device. In such a case, the display state setting section 114 (see FIG. 5, for example) functions as an alert information output section.

When the first image acquisition section 110 has acquired the first image, the second image acquisition section 111 has acquired the second image, and the attention area detection section 112 has detected an attention area based on the feature quantity of each pixel within the second image, the alert information output section (display state setting section 114) may output the alert information about the detected attention area. More specifically, the designated elapsed time setting section 113 may perform the designated elapsed time setting process based on the attention area detection result, and the alert information output section may output the alert information until the designated elapsed time elapses. When using sound as the alert information, the display section 115 (see FIG. 5, for example) functions as a sound output section. The sound output section may output alert sound that notifies the user of detection of an attention area as the alert information until the designated elapsed time elapses after the attention area has been detected. According to this configuration, the user can determine that the attention area is present near the current observation position even when the attention area is detected only for a short time. This makes it possible to prevent a situation in which the attention area is missed, and reliably specify the attention area.

Although only some embodiments of the invention have been described in detail above, those skilled in the art would readily appreciate that many modifications are possible in the embodiments without materially departing from the novel teachings and advantages of the invention. Accordingly, such modifications are intended to be included within the scope of the invention. Any term (e.g., normal light image or special light image) cited with a different term (e.g., first image or second image) having a broader meaning or the same meaning at least once in the specification and the drawings may be replaced by the different term in any place in the specification and the drawings. The configurations and the operations of the image processing device and the endoscope system are not limited to those described in connection with the above embodiments. Various modifications and variations may be made of the above embodiments, within the scope of the claims.

REFERENCE SIGNS LIST

90: image processing device, 100: lens system, 101: CCD, 102: illumination light source, 103: rotary filter, 104:

gain amplifier, 105: A/D conversion section, 106: buffer, 107: WB section, 108: photometrical evaluation section, 109: switch section, 110: first image acquisition section, 111: second image acquisition section, 112: attention area detection section, 113: designated elapsed time setting section, 114: display state setting section, 115: display section (output section), 116: control section, 117: external I/F section, 200: buffer, 201: hue/chroma calculation section, 202: attention area determination section, 203: threshold value ROM, 204: buffer, 205: reliability calculation section, 206: area determination section, 300: update section, 301: detection information recording section, 302: control information output section, 303: buffer, 304: motion amount detection section, 305: designated elapsed time calculation section, 306: designated elapsed time ROM, 398: processing section, 400: processing section, 401: selection section, 402: alert information addition section, 403: luminance/color difference separation section, 404: edge enhancement section, 405: chroma enhancement section, 406: luminance/color difference blending section, 410: buffer, 500: designated elapsed time setting section, 501: display state setting section, 550: CCD, 551: rotary filter, 552: switch section, 600: computer system, 610: main body, 611: CPU, 612: RAM, 613: ROM, 614: HDD, 615: CD-ROM drive, 616: USB port, 617: I/O interface, 618: LAN interface, 620: display, 621: display screen, 630: keyboard, 640: mouse, 650: modem, 660: CD-ROM, 670: USB memory, 681: PC, 682: server, 683: printer

## Claims

1. An image processing device (90) comprising:

    a first image acquisition section (110) that acquires a first image, the first image being an image that has information within a wavelength band of white light; and
    a second image acquisition section (111) that acquires a second image, the second image being an image that has information within a specific wavelength band,
    an attention area detection section (112) that detects an attention area within the second image based on a feature quantity of each pixel within the second image;
    a display state setting section (114) that performs a display state setting process that sets a display state of a display image generated based on the first image; **characterized in that** the image processing device (90) further comprises a designated elapsed time setting section (113) that performs a designated elapsed time setting process that sets a designated elapsed time based on a detection result for the attention area, wherein the display state setting section (114) is adapted to perform the display state setting process based on the designated elapsed time that has been set by the designated elapsed time setting section (113).

2. The image processing device (90) as defined in claim 1,
the display state setting section (114) performing the display state setting process that changes the display state of the display image until the designated elapsed time elapses.

3. The image processing device (90) as defined in claim 2,
the display state setting section (114) performing the display state setting process that displays the display image on which alert information about the attention area is set, until the designated elapsed time elapses.

4. The image processing device (90) as defined in claim 2,
the display state setting section (114) performing the display state setting process that changes the display state of the display image until the designated elapsed time elapses even when the attention area has not been detected within the second image within the designated elapsed time.

5. The image processing device (90) as defined in claim 4,
the designated elapsed time setting section (113) setting a new designated elapsed time starting from a detection timing of the attention area, when the attention area has been detected within the second image within the designated elapsed time.

6. The image processing device (90) as defined in claim 1,
the first image acquisition section (110) acquiring the first image corresponding to at least one frame every cycle period,
the second image acquisition section (111) acquiring the second image corresponding to at least one frame every cycle period, and
the display state setting section (114) performing the display state setting process that changes the display state of the display image in an (N+1)th (N is a natural number) cycle period subsequent to an Nth cycle period when the attention area has been detected within the second image in the Nth cycle period.

7. The image processing device (90) as defined in claim 6,
the first image acquisition section (110) acquiring the first images corresponding to K frames (K is a natural number) every cycle period,
the second image acquisition section (111) acquiring the second images corresponding to L frames (L is a natural number) every cycle period, and
a relationship "K>L" being satisfied.

8. The image processing device (90) as defined in claim 7,
the first image acquisition section (110) acquiring the first images corresponding to the K frames every cycle period, and
the second image acquisition section (111) acquiring the second image corresponding to one frame every cycle period.

9. The image processing device (90) as defined in claim 6,
a relationship "TE>T" being satisfied when a length of each cycle period is referred to as T, and a length of the designated elapsed time is referred to as TE.

10. The image processing device (90) as defined in claim 6,
the display state setting section (114) performing the display state setting process that changes the display state of the display image in (N+1)th to Mth cycle periods (M is an integer that satisfies "M>N+1") when the attention area has been detected within the second image in the Nth cycle period, and the attention area has not been detected within the second image in the (N+1)th cycle period.

11. The image processing device (90) as defined in claim 1,
the designated elapsed time setting section including:

a detection information recording section (301) that records detection information about the attention area;
an update section (300) that performs an update process that updates the detection information based on the detection result from the attention area detection section (112); and
a control information output section (302) that outputs control information that controls the display state setting process performed by the display state setting section (114) based on the detection information recorded in the detection information recording section (301).

12. The image processing device (90) as defined in claim 11,
the update section (300) setting a value VD of the detection information to a first value VD1 that corresponds to the designated elapsed time when the attention area has been detected within the second image, and performing the update process that sequentially changes the value VD of the detection information from the first value VD1 to a second value VD2 when the attention area has not been detected within the second image, and
the control information output section (302) outputting the control information that instructs the display state setting section (114) to change the display state of the display image until the value VD of the detection information reaches the second value VD2, and outputting the control information that instructs the display state setting section (114) not to change the display state of the display image when the value VD of the detection information has reached the second value VD2.

13. The image processing device (90) as defined in claim 11,
the first image acquisition section (110) acquiring the first image corresponding to at least one frame every cycle period,
the second image acquisition section (111) acquiring the second image corresponding to at least one frame every cycle period, and
the update section (300) performing the update process that updates the detection information every cycle period.

14. The image processing device (90) as defined in claim 13,
the update section (300) setting a value VD of the detection information to a first value VD1 that corresponds to the designated elapsed time when the attention area has been detected within the second image in an Nth (N is a natural number) cycle period, and performing the update process that sequentially changes the value VD of the detection information from the first value VD1 to a second value VD2 when the attention area has not been detected within the second image in a cycle period subsequent to the Nth cycle period, and

the control information output section (302) outputting the control information that instructs the display state setting section (114) to change the display state of the display image until the value VD of the detection information reaches the second value VD2, and outputting the control information that instructs the display state setting section (114) not to change the display state of the display image when the value VD of the detection information has reached the second value VD2.

15. The image processing device (90) as defined in claim 14,
the update section (300) resetting the value VD of the detection information to the first value VD1 again when the attention area has been detected within the second image in a cycle period subsequent to the Nth cycle period.

16. The image processing device (90) as defined in claim 11,
the display state setting section (114) including a processing section (400),
the processing section (400) processing the first image, and outputting the processed first image as the display image when the control information from the control information output section (302) instructs to change the display state of the display image, and directly outputting the first image as the display image when the control information from the control information output section (302) instructs not to change the display state of the display image.

17. The image processing device (90) as defined in claim 1,
the attention area detection section (112) including a reliability calculation section (205) that calculates reliability of the attention area detected by the attention area detection section (112).

18. The image processing device (90) as defined in claim 17,
the reliability calculation section (205) calculating the reliability based on an area of the attention area detected by the attention area detection section (112).

19. The image processing device (90) as defined in claim 1,
the designated elapsed time setting section (113) including a motion amount detection section (304) that detects a motion amount of the first image, and
the designated elapsed time setting section (113) performing the designated elapsed time setting process based on the motion amount detected by the motion amount detection section (304).

20. The image processing device (90) as defined in claim 19,
the designated elapsed time setting section (113) performing the designated elapsed time setting process that reduces the designated elapsed time as the motion amount increases.

21. The image processing device (90) as defined in claim 19,
the motion amount detection section (304) detecting the motion amount of the first image when the attention area has been detected within the second image.

22. The image processing device (90) as defined in claim 19,
the designated elapsed time setting section (113) including:

a detection information recording section (301) that records detection information about the attention area;
an update section (300) that performs an update process that updates the detection information based on the detection result from the attention area detection section (112); and
a control information output section (302) that outputs control information that controls the display state setting process performed by the display state setting section (114) based on the detection information recorded in the detection information recording section (301), and
the update section (300) setting a value of the detection information based on the motion amount detected by the motion amount detection section (304).

23. The image processing device (90) as defined in claim 22,
the update section (300) setting a value VD of the detection information to a first value VD1 that changes depending on the motion amount detected by the motion amount detection section (304) when the attention area has been detected within the second image, and performing the update process that sequentially changes the value VD of the detection information from the first value VD1 to a second value VD2 when the attention area has not been detected within the second image, and

the control information output section (302) outputting the control information that instructs the display state setting section (114) to change the display state of the display image until the value VD of the detection information reaches the second value VD2, and outputting the control information that instructs the display state setting section (114) not to change the display state of the display image when the value VD of the detection information has reached the second value VD2.

24. The image processing device (90) as defined in claim 1,
the display state setting section (114) performing the display state setting process that enhances at least one of an edge and a chroma of the first image.

25. The image processing device (90) as defined in claim 1,
the specific wavelength band being narrower than the wavelength band of the white light.

26. The image processing device (90) as defined in claim 1,
the first image and the second image being an *in vivo* image, and
the specific wavelength band included in the *in vivo* image being a wavelength band of light absorbed by hemoglobin in blood.

27. The image processing device (90) as defined in claim 26,
the specific wavelength band being 390 to 445 nm or 530 to 550 nm.

28. The image processing device (90) as defined in claim 1,
the first image and the second image being an *in vivo* image, and
the specific wavelength band included in the *in vivo* image being a wavelength band of fluorescence produced by a fluorescent substance.

29. The image processing device (90) as defined in claim 28,
the specific wavelength band being 490 to 625 nm.

30. The image processing device (90) as defined in claim 1,
the first image and the second image being an *in vivo* image, and
the specific wavelength band included in the *in vivo* image being a wavelength band of infrared light.

31. The image processing device (90) as defined in claim 30,
the specific wavelength band being 790 to 820 nm or 905 to 970 nm.

32. The image processing device (90) as defined in claim 1,
the second image acquisition section (111) generating the second image based on the first image acquired by the first image acquisition section (110).

33. The image processing device (90) as defined in claim 32,
the second image acquisition section (111) including a signal extraction section that extracts a signal within the wavelength band of the white light from the first image acquired by the first image acquisition section (110), and
the second image acquisition section (111) generating the second image that includes a signal within the specific wavelength band based on the signal within the wavelength band of the white light extracted by the signal extraction section.

34. The image processing device (90) as defined in claim 33,
the second image acquisition section (111) including a matrix data setting section that sets matrix data for calculating the signal within the specific wavelength band from the signal within the wavelength band of the white light, and
the second image acquisition section (111) calculating the signal within the specific wavelength band from the signal within the wavelength band of white light using the matrix data set by the matrix data setting section to generate the second image.

35. A program that causes a computer to function as:

a first image acquisition section (110) that acquires a first image, the first image being an image that has information within a wavelength band of white light; and

a second image acquisition section (111) that acquires a second image, the second image being an image that has information within a specific wavelength band,
an attention area detection section (112) that detects an attention area within the second image based on a feature quantity of each pixel within the second image;
a display state setting section (114) that performs a display state setting process that sets a display state of a display image generated based on the first image; **characterized in that** the program causes the computer to further function as a designated elapsed time setting section (113) that performs a designated elapsed time setting process that sets a designated elapsed time based on a detection result for the attention area,
the display state setting section (114) performing the display state setting process based on the designated elapsed time that has been set by the designated elapsed time setting section (113).

**Patentansprüche**

1. Bildverarbeitungsvorrichtung (90), die umfasst:

einen ersten Bilderfassungsabschnitt (110), der ein erstes Bild erfasst, wobei das erste Bild ein Bild ist, das Informationen innerhalb eines Wellenlängenbands von Weißlicht aufweist; und
einen zweiten Bilderfassungsabschnitt (111), der ein zweites Bild erfasst, wobei das zweite Bild ein Bild ist, das Informationen innerhalb eines spezifischen Wellenlängenbands aufweist,
einen Aufmerksamkeitsbereicherkennungsabschnitt (112), der einen Aufmerksamkeitsbereich innerhalb des zweiten Bilds auf Basis einer Merkmalsmenge jedes Pixels innerhalb des zweiten Bilds erkennt;
einen Anzeigestatuseinstellabschnitt (114), der einen Anzeigestatuseinstellprozess durchführt, der einen Anzeigestatus eines Anzeigebilds einstellt, das auf Basis des ersten Bilds erzeugt wurde; **dadurch gekennzeichnet, dass** die Bildverarbeitungsvorrichtung (90) ferner umfasst:
einen Abschnitt (113) zum Einstellen eines designierten verstrichenen Zeitraums, der einen Prozess zum Einstellen eines designierten verstrichenen Zeitraums durchführt, der einen designierten verstrichenen Zeitraum auf Basis eines Erkennungsergebnisses für den Aufmerksamkeitsbereich einstellt;
wobei der Anzeigestatuseinstellabschnitt (114) so ausgelegt ist, dass er den Anzeigestatuseinstellprozess auf Basis des designierten verstrichenen Zeitraums durchführt, der vom Abschnitt (113) zum Einstellen eines designierten verstrichenen Zeitraums eingestellt wurde.

2. Bildverarbeitungsvorrichtung (90) nach Anspruch 1,
wobei der Anzeigestatuseinstellabschnitt (114) den Anzeigestatuseinstellprozess durchführt, der den Anzeigestatus des Anzeigebilds ändert, bis der designierte verstrichene Zeitraum verstrichen ist.

3. Bildverarbeitungsvorrichtung (90) nach Anspruch 2,
wobei der Anzeigestatuseinstellabschnitt (114) den Anzeigestatuseinstellprozess durchführt, der das Anzeigebild anzeigt, in Bezug auf welches Warninformationen zum Aufmerksamkeitsbereich eingestellt sind, bis der designierte verstrichene Zeitraum verstrichen ist.

4. Bildverarbeitungsvorrichtung (90) nach Anspruch 2,
wobei der Anzeigestatuseinstellabschnitt (114) den Anzeigestatuseinstellprozess, der den Anzeigestatus des Anzeigebilds ändert, bis der designierte verstrichene Zeitraum verstrichen ist, sogar dann durchführt, wenn der Aufmerksamkeitsbereich innerhalb des zweiten Bilds innerhalb des designierten verstrichenen Zeitraums nicht erkannt wurde.

5. Bildverarbeitungsvorrichtung (90) nach Anspruch 4,
wobei der Abschnitt (113) zum Einstellen eines designierten verstrichenen Zeitraums einen neuen designierten verstrichenen Zeitraum mit Beginn von einem Erkennungszeitpunkt des Aufmerksamkeitsbereichs einstellt, wenn der Aufmerksamkeitsbereich innerhalb des zweiten Bilds innerhalb des designierten verstrichenen Zeitraums erkannt wurde.

6. Bildverarbeitungsvorrichtung (90) nach Anspruch 1,
wobei der erste Bilderfassungsabschnitt (110) das erste Bild, das zumindest einem Frame entspricht, jede Zyklusperiode erfasst,
wobei der zweite Bilderfassungsabschnitt (111) das zweite Bild, das zumindest einem Frame entspricht, jede Zyklusperiode erfasst, und

wobei der Anzeigestatuseinstellabschnitt (114) den Anzeigestatuseinstellprozess, der den Anzeigestatus des Anzeigebilds ändert, in einer (N + 1)-ten (N ist eine natürliche Zahl) Zyklusperiode nach einer N-ten Zyklusperiode durchführt, wenn der Aufmerksamkeitsbereich innerhalb des zweiten Bilds in der N-ten Zyklusperiode erkannt wurde.

7. Bildverarbeitungsvorrichtung (90) nach Anspruch 6,
wobei der erste Bilderfassungsabschnitt (110) die ersten Bilder, die K Frames (K ist eine natürliche Zahl) entsprechen, jede Zyklusperiode erfasst,
wobei der zweite Bilderfassungsabschnitt (111) die zweiten Bilder, die L Frames (L ist eine natürliche Zahl) entsprechen, jede Zyklusperiode erfasst und
eine Beziehung "K > L" erfüllt ist.

8. Bildverarbeitungsvorrichtung (90) nach Anspruch 7,
wobei der erste Bilderfassungsabschnitt (110) die ersten Bilder, die den K Frames entsprechen, jede Zyklusperiode erfasst, und
wobei der zweite Bilderfassungsabschnitt (111) das zweite Bild, das einem Frame entspricht, jede Zyklusperiode erfasst.

9. Bildverarbeitungsvorrichtung (90) nach Anspruch 6,
wobei eine Beziehung "TE > T" erfüllt ist, wenn eine Länge jeder Zyklusperiode als T bezeichnet wird und eine Länge des designierten verstrichenen Zeitraums als TE bezeichnet wird.

10. Bildverarbeitungsvorrichtung (90) nach Anspruch 6,
wobei der Anzeigestatuseinstellabschnitt (114) den Anzeigestatuseinstellprozess, der den Anzeigestatus des Anzeigebilds ändert, in (N + 1)-ten bis M-ten Zyklusperioden (M ist eine ganze Zahl, die "M > N + 1" erfüllt) durchführt, wenn der Aufmerksamkeitsbereich innerhalb des zweiten Bilds in der N-ten Zyklusperiode erkannt wurde und der Aufmerksamkeitsbereich innerhalb des zweiten Bilds in der (N + 1)-ten Zyklusperiode nicht erkannt wurde.

11. Bildverarbeitungsvorrichtung (90) nach Anspruch 1,
wobei der Abschnitt zum Einstellen eines designierten verstrichenen Zeitraums umfasst:

einen Erkennungsinformationsaufzeichnungsabschnitt (301), der Erkennungsinformationen zum Aufmerksamkeitsbereich aufzeichnet;
einen Aktualisierungsabschnitt (300), der einen Aktualisierungsprozess durchführt, der die Erkennungsinformationen auf Basis des Erkennungsergebnisses vom Aufmerksamkeitserkennungsabschnitt (112) aktualisiert; und
einen Steuerinformationsausgabeabschnitt (302), der Steuerinformationen ausgibt, die den vom Anzeigestatuseinstellabschnitt (114) durchgeführten Anzeigestatuseinstellprozess auf Basis der im Erkennungsinformationsaufzeichnungsabschnitt (301) aufgezeichneten Erkennungsinformationen steuern.

12. Bildverarbeitungsvorrichtung (90) nach Anspruch 11,
wobei der Aktualisierungsabschnitt (300) einen Wert VD der Erkennungsinformationen auf einen ersten Wert VD1 einstellt, der dem designierten verstrichenen Zeitraum entspricht, wenn der Aufmerksamkeitsbereich innerhalb des zweiten Bilds erkannt wurde, und den Aktualisierungsprozess durchführt, der den Wert VD der Erkennungsinformationen vom ersten Wert VD1 sequentiell auf einen zweiten Wert VD2 ändert, wenn der Aufmerksamkeitsbereich innerhalb des zweiten Bilds nicht erkannt wurde, und
wobei der Steuerinformationsausgabeabschnitt (302) die Steuerinformationen ausgibt, die den Anzeigestatuseinstellabschnitt (114) anweisen, den Anzeigestatus des Anzeigebilds zu ändern, bis der Wert VD der Erkennungsinformationen den zweiten Wert VD2 erreicht hat, und die Steuerinformationen ausgibt, die den Anzeigestatuseinstellabschnitt (114) anweisen, den Anzeigestatus des Anzeigebilds nicht zu ändern, wenn der Wert VD der Erkennungsinformationen den zweiten Wert VD2 erreicht hat.

13. Bildverarbeitungsvorrichtung (90) nach Anspruch 11,
wobei der erste Bilderfassungsabschnitt (110) das erste Bild, das zumindest einem Frame entspricht, jede Zyklusperiode erfasst,
wobei der zweite Bilderfassungsabschnitt (111) das zweite Bild, das zumindest einem Frame entspricht, jede Zyklusperiode erfasst, und
wobei der Aktualisierungsabschnitt (300) den Aktualisierungsprozess, der die Erkennungsinformationen aktualisiert, jede Zyklusperiode durchführt.

**14.** Bildverarbeitungsvorrichtung (90) nach Anspruch 13,
wobei der Aktualisierungsabschnitt (300) einen Wert VD der Erkennungsinformationen auf einen ersten Wert VD1 einstellt, der dem designierten verstrichenen Zeitraum entspricht, wenn der Aufmerksamkeitsbereich innerhalb des zweiten Bilds in einer N-ten (N ist eine natürliche Zahl) Zyklusperiode erkannt wurde, und den Aktualisierungsprozess durchführt, der den Wert VD der Erkennungsinformationen vom ersten Wert VD1 sequentiell auf einen zweiten Wert VD2 ändert, wenn der Aufmerksamkeitsbereich innerhalb des zweiten Bilds in einer Zyklusperiode nach der N-ten Zyklusperiode nicht erkannt wurde, und
wobei der Steuerinformationsausgabeabschnitt (302) die Steuerinformationen ausgibt, die den Anzeigestatuseinstellabschnitt (114) anweisen, den Anzeigestatus des Anzeigebilds zu ändern, bis der Wert VD der Erkennungsinformationen den zweiten Wert VD2 erreicht hat, und die Steuerinformationen ausgibt, die den Anzeigestatuseinstellabschnitt (114) anweisen, den Anzeigestatus des Anzeigebilds nicht zu ändern, wenn der Wert VD der Erkennungsinformationen den zweiten Wert VD2 erreicht hat.

**15.** Bildverarbeitungsvorrichtung (90) nach Anspruch 14,
wobei der Aktualisierungsabschnitt (300) den Wert VD der Erkennungsinformationen wieder auf den ersten Wert VD1 zurücksetzt, wenn der Aufmerksamkeitsbereich innerhalb des zweiten Bilds in einer Zyklusperiode nach der N-ten Zyklusperiode erkannt wurde.

**16.** Bildverarbeitungsvorrichtung (90) nach Anspruch 11,
wobei der Anzeigestatuseinstellabschnitt (114) einen Verarbeitungsabschnitt (400) umfasst, wobei der Verarbeitungsabschnitt (400) das erste Bild verarbeitet und das verarbeitete erste Bild als Anzeigebild ausgibt, wenn die Steuerinformationen vom Steuerinformationsausgabeabschnitt (302) eine Änderung des Anzeigestatus des Anzeigebilds anweisen, und das erste Bild direkt als Anzeigebild ausgibt, wenn die Steuerinformationen vom Steuerinformationsausgabeabschnitt (302) keine Änderung des Anzeigestatus des Anzeigebilds anweisen.

**17.** Bildverarbeitungsvorrichtung (90) nach Anspruch 1,
wobei der Aufmerksamkeitserkennungsabschnitt (112) einen Zuverlässigkeitsberechnungsabschnitt (205) umfasst, der eine Zuverlässigkeit des vom Aufmerksamkeitsbereichserkennungsabschnitts (112) erkannten Aufmerksamkeitsbereichs berechnet.

**18.** Bildverarbeitungsvorrichtung (90) nach Anspruch 17,
wobei der Zuverlässigkeitsberechnungsabschnitt (205) die Zuverlässigkeit auf Basis eines Bereichs des vom Aufmerksamkeitsbereichserkennungsabschnitts (112) erkannten Aufmerksamkeitsbereichs berechnet.

**19.** Bildverarbeitungsvorrichtung (90) nach Anspruch 1,
wobei der Abschnitt (113) zum Einstellen eines designierten verstrichenen Zeitraums einen Bewegungsausmaßerkennungsabschnitt (304) umfasst, der ein Bewegungsausmaß des ersten Bilds erkennt, und
wobei der Abschnitt (113) zum Einstellen eines designierten verstrichenen Zeitraums den Prozess zum Einstellen eines designierten verstrichenen Zeitraums auf Basis des vom Bewegungsausmaßerkennungsabschnitt (304) erkannten Bewegungsausmaßes durchführt.

**20.** Bildverarbeitungsvorrichtung (90) nach Anspruch 19,
wobei der Abschnitt (113) zum Einstellen eines designierten verstrichenen Zeitraums den Prozess zum Einstellen eines designierten verstrichenen Zeitraums durchführt, der den designierten verstrichenen Zeitraum mit steigendem Bewegungsausmaß verkürzt.

**21.** Bildverarbeitungsvorrichtung (90) nach Anspruch 19,
wobei der Bewegungsausmaßerkennungsabschnitt (304) das Bewegungsausmaß des ersten Bilds erkennt, wenn der Aufmerksamkeitsbereich innerhalb des zweiten Bilds erkannt wurde.

**22.** Bildverarbeitungsvorrichtung (90) nach Anspruch 19,
wobei der Abschnitt (113) zum Einstellen eines designierten verstrichenen Zeitraums umfasst:

einen Erkennungsinformationsaufzeichnungsabschnitt (301), der Erkennungsinformationen zum Aufmerksamkeitsbereich aufzeichnet;
einen Aktualisierungsabschnitt (300), der einen Aktualisierungsprozess durchführt, der die Erkennungsinformationen auf Basis des Erkennungsergebnisses vom Aufmerksamkeitserkennungsabschnitt (112) aktualisiert; und

einen Steuerinformationsausgabeabschnitt (302), der Steuerinformationen ausgibt, die den vom Anzeigestatuseinstellabschnitt (114) durchgeführten Anzeigestatuseinstellprozess auf Basis der im Erkennungsinformationsaufzeichnungsabschnitt (301) aufgezeichneten Erkennungsinformationen steuern, und

wobei der Aktualisierungsabschnitt (300) die Erkennungsinformationen auf Basis des vom Bewegungsausmaßerkennungsabschnitt (304) erkannten Bewegungsausmaßes einstellt.

23. Bildverarbeitungsvorrichtung (90) nach Anspruch 22,
wobei der Aktualisierungsabschnitt (300) einen Wert VD der Erkennungsinformationen auf einen ersten Wert VD1 einstellt, der sich je nach dem vom Bewegungsausmaßerkennungsabschnitt (304) erkannten Bewegungsausmaß ändert, wenn der Aufmerksamkeitsbereich innerhalb des zweiten Bilds erkannt wurde, und den Aktualisierungsprozess durchführt, der den Wert VD der Erkennungsinformationen vom ersten Wert VD1 sequentiell auf einen zweiten Wert VD2 ändert, wenn der Aufmerksamkeitsbereich innerhalb des zweiten Bilds nicht erkannt wurde, und wobei der Steuerinformationsausgabeabschnitt (302) die Steuerinformationen ausgibt, die den Anzeigestatuseinstellabschnitt (114) anweisen, den Anzeigestatus des Anzeigebilds zu ändern, bis der Wert VD der Erkennungsinformationen den zweiten Wert VD2 erreicht hat, und die Steuerinformationen ausgibt, die den Anzeigestatuseinstellabschnitt (114) anweisen, den Anzeigestatus des Anzeigebilds nicht zu ändern, wenn der Wert VD der Erkennungsinformationen den zweiten Wert VD2 erreicht hat.

24. Bildverarbeitungsvorrichtung (90) nach Anspruch 1,
wobei der Anzeigestatuseinstellabschnitt (114) den Anzeigestatuseinstellprozess durchführt, der zumindest eines von einem Rand und einer Farbsättigung des ersten Bilds verbessert.

25. Bildverarbeitungsvorrichtung (90) nach Anspruch 1,
wobei das spezifische Wellenlängenband schmäler als das Wellenlängenband des Weißlichts ist.

26. Bildverarbeitungsvorrichtung (90) nach Anspruch 1,
wobei das erste Bild und das zweite Bild ein *In-vivo*-Bild sind und das im *In-vivo*-Bild umfasste spezifische Wellenlängenband ein Wellenlängenband von Licht ist, das von Hämoglobin im Blut absorbiert wird.

27. Bildverarbeitungsvorrichtung (90) nach Anspruch 26,
wobei das spezifische Wellenlängenband 390 bis 445 nm oder 530 bis 550 nm ist.

28. Bildverarbeitungsvorrichtung (90) nach Anspruch 1,
wobei das erste Bild und das zweite Bild ein *In-vivo*-Bild sind und das im *In-vivo*-Bild umfasste spezifische Wellenlängenband ein Wellenlängenband von Fluoreszenz ist, die von einer fluoreszierenden Substanz erzeugt wird.

29. Bildverarbeitungsvorrichtung (90) nach Anspruch 28,
wobei das spezifische Wellenlängenband 490 bis 625 nm ist.

30. Bildverarbeitungsvorrichtung (90) nach Anspruch 1,
wobei das erste Bild und das zweite Bild ein *In-vivo*-Bild sind und das im *In-vivo*-Bild umfasste spezifische Wellenlängenband ein Wellenlängenband von InfrarotLicht ist.

31. Bildverarbeitungsvorrichtung (90) nach Anspruch 30,
wobei das spezifische Wellenlängenband 790 bis 820 nm oder 905 bis 970 nm ist.

32. Bildverarbeitungsvorrichtung (90) nach Anspruch 1,
wobei der zweite Bilderfassungsabschnitt (111) das zweite Bild auf Basis des vom ersten Bilderfassungsabschnitt (110) erfassten ersten Bilds erzeugt.

33. Bildverarbeitungsvorrichtung (90) nach Anspruch 32,
wobei der zweite Bilderfassungsabschnitt (111) einen Signalextraktionsabschnitt umfasst, der ein Signal innerhalb des Wellenlängenbands des Weißlichts vom ersten Bild extrahiert, das vom ersten Bilderfassungsabschnitt (110) erfasst wurde, und wobei der zweite Bilderfassungsabschnitt (111) das zweite Bild erzeugt, das ein Signal innerhalb des spezifischen Wellenlängenbands umfasst, auf Basis des Signals innerhalb des Wellenlängenbands des Weißlichts, wie vom Signalextraktionsabschnitt extrahiert.

**34.** Bildverarbeitungsvorrichtung (90) nach Anspruch 33,
wobei der zweite Bilderfassungsabschnitt (111) einen Matrixdateneinstellabschnitt umfasst, der Matrixdaten zum Berechnen des Signals innerhalb des spezifischen Wellenlängenbands vom Signal innerhalb des Wellenlängenbands des Weißlichts einstellt, und
der zweite Bilderfassungsabschnitt (111) das Signal innerhalb des spezifischen Wellenlängenbands vom Signal innerhalb des Wellenlängenbands von Weißlicht unter Verwendung der Matrixdaten berechnet, die vom Matrixdateneinstellabschnitt eingestellt wurden, um das zweite Bild zu erzeugen.

**35.** Programm, das einen Computer dazu veranlasst, zu agieren als:

ein erster Bilderfassungsabschnitt (110), der ein erstes Bild erfasst, wobei das erste Bild ein Bild ist, das Informationen innerhalb eines Wellenlängenbands von Weißlicht aufweist; und
ein zweiter Bilderfassungsabschnitt (111), der ein zweites Bild erfasst, wobei das zweite Bild ein Bild ist, das Informationen innerhalb eines spezifischen Wellenlängenbands aufweist,
ein Aufmerksamkeitsbereicherkennungsabschnitt (112), der einen Aufmerksamkeitsbereich innerhalb des zweiten Bilds auf Basis einer Merkmalsmenge jedes Pixels innerhalb des zweiten Bilds erkennt;
ein Anzeigestatuseinstellabschnitt (114), der einen Anzeigestatuseinstellprozess durchführt, der einen Anzeigestatus eines Anzeigebilds einstellt, das auf Basis des ersten Bilds erzeugt wurde; **dadurch gekennzeichnet, dass** das Programm den Computer dazu veranlasst, ferner zu agieren als:
ein Abschnitt (113) zum Einstellen eines designierten verstrichenen Zeitraums, der einen Prozess zum Einstellen eines designierten verstrichenen Zeitraums durchführt, der einen designierten verstrichenen Zeitraum auf Basis eines Erkennungsergebnisses für den Aufmerksamkeitsbereich einstellt,
wobei der Anzeigestatuseinstellabschnitt (114) den Anzeigestatuseinstellprozess auf Basis des designierten verstrichenen Zeitraums durchführt, der vom Abschnitt (113) zum Einstellen eines designierten verstrichenen Zeitraums eingestellt wurde.

**Revendications**

**1.** Dispositif de traitement d'images (90) comprenant :

une première section d'acquisition d'image (110) qui acquiert une première image, la première image étant une image qui possède des informations à l'intérieur d'une bande de longueur d'onde de lumière blanche ; et
une seconde section d'acquisition d'image (111) qui acquiert une seconde image, la seconde image étant une image qui possède des informations à l'intérieur d'une bande de longueur d'onde spécifique,
une section de détection de zone d'attention (112) qui détecte une zone d'attention à l'intérieur de la seconde image sur la base d'une quantité caractéristique de chaque pixel à l'intérieur de la seconde image ;
une section de réglage d'état d'affichage (114) qui réalise un processus de réglage d'état d'affichage qui règle un état d'affichage d'une image d'affichage générée sur la base de la première image ;
**caractérisé par le fait que** le dispositif de traitement d'images (90) comprend en outre :

une section de réglage de temps écoulé désigné (113) qui réalise un processus de réglage de temps écoulé désigné qui règle un temps écoulé désigné sur la base d'un résultat de détection pour la zone d'attention,
dans lequel la section de réglage d'état d'affichage (114) est conçue pour réaliser le processus de réglage d'état d'affichage sur la base du temps écoulé désigné qui a été réglé par la section de réglage de temps écoulé désigné (113).

**2.** Dispositif de traitement d'images (90) tel que défini à la revendication 1,
la section de réglage d'état d'affichage (114) réalisant le processus de réglage d'état d'affichage qui modifie l'état d'affichage de l'image d'affichage jusqu'à ce que le temps écoulé désigné s'écoule.

**3.** Dispositif de traitement d'images (90) tel que défini à la revendication 2,
la section de réglage d'état d'affichage (114) réalisant le processus de réglage d'état d'affichage qui affiche l'image d'affichage sur laquelle des informations d'alerte concernant la zone d'attention sont réglées, jusqu'à ce que le temps écoulé désigné s'écoule.

**4.** Dispositif de traitement d'images (90) tel que défini à la revendication 2,
la section de réglage d'état d'affichage (114) réalisant le processus de réglage d'état d'affichage qui modifie l'état d'affichage de l'image d'affichage jusqu'à ce que le temps écoulé désigné s'écoule même lorsque la zone d'attention n'a pas été détectée à l'intérieur de la seconde image dans le temps écoulé désigné.

5. Dispositif de traitement d'images (90) tel que défini à la revendication 4, la section de réglage de temps écoulé désigné (113) réglant un nouveau temps écoulé désigné démarrant à partir d'une temporisation de détection de la zone d'attention, lorsque la zone d'attention a été détectée à l'intérieur de la seconde image dans le temps écoulé désigné.

6. Dispositif de traitement d'images (90) tel que défini à la revendication 1, la première section d'acquisition d'image (110) acquérant la première image correspondant à au moins une trame chaque période de cycle, la seconde section d'acquisition d'image (111) acquérant la seconde image correspondant à au moins une trame chaque période de cycle, et la section de réglage d'état d'affichage (114) réalisant le processus de réglage d'état d'affichage qui modifie l'état d'affichage de l'image d'affichage dans une (N+1)ième (N est un nombre naturel) période de cycle qui suit une Nième période de cycle lorsque la zone d'attention a été détectée à l'intérieur de la seconde image dans la Nième période de cycle.

7. Dispositif de traitement d'images (90) tel que défini à la revendication 6, la première section d'acquisition d'image (110) acquérant les premières images correspondant à K trames (K est un nombre naturel) chaque période de cycle, la seconde section d'acquisition d'image (111) acquérant les secondes images correspondant à L trames (L est un nombre naturel) chaque période de cycle, et une relation « K > L » étant satisfaite.

8. Dispositif de traitement d'images (90) tel que défini à la revendication 7, la première section d'acquisition d'image (110) acquérant les premières images correspondant aux K trames chaque période de cycle, et la seconde section d'acquisition d'image (111) acquérant la seconde image correspondant à une trame chaque période de cycle.

9. Dispositif de traitement d'images (90) tel que défini à la revendication 6, une relation « TE > T » étant satisfaite lorsqu'une longueur de chaque période de cycle est référencée en tant que T, et qu'une longueur du temps écoulé désigné est référencée en tant que TE.

10. Dispositif de traitement d'images (90) tel que défini à la revendication 6, la section de réglage d'état d'affichage (114) réalisant le processus de réglage d'état d'affichage qui modifie l'état d'affichage de l'image d'affichage dans des (N+1)ième à Mième périodes de cycle (M est un nombre entier qui satisfait à « M > N+1 ») lorsque la zone d'attention a été détectée à l'intérieur de la seconde image dans la Nième période de cycle, et que la zone d'attention n'a pas été détectée à l'intérieur de la seconde image dans la (N+1)ième période de cycle.

11. Dispositif de traitement d'images (90) tel que défini à la revendication 1, la section de réglage de temps écoulé désigné comprenant :

une section d'enregistrement d'informations de détection (301) qui enregistre des informations de détection concernant la zone d'attention ;
une section de mise à jour (300) qui réalise un processus de mise à jour qui met à jour les informations de détection sur la base du résultat de détection en provenance de la section de détection de zone d'attention (112) ; et
une section de délivrance d'informations de commande (302) qui délivre des informations de commande qui commande le processus de réglage d'état d'affichage réalisé par la section de réglage d'état d'affichage (114) sur la base des informations de détection enregistrées dans la section d'enregistrement d'informations de détection (301).

12. Dispositif de traitement d'images (90) tel que défini à la revendication 11, la section de mise à jour (300) réglant une valeur VD des informations de détection à une première valeur VD1 qui correspond au temps écoulé désigné lorsque la zone d'attention a été détectée à l'intérieur de la seconde image, et réalisant le processus de mise à jour qui modifie séquentiellement la valeur VD des informations de détection depuis la première valeur VD1 vers une seconde valeur VD2 lorsque la zone d'attention n'a pas été détectée à l'intérieur de la seconde image, et

la section de délivrance d'informations de commande (302) délivrant les informations de commande qui donnent l'instruction à la section de réglage d'état d'affichage (114) de modifier l'état d'affichage de l'image d'affichage jusqu'à ce que la valeur VD des informations de détection atteigne la seconde valeur VD2, et délivrant les informations de commande qui donnent l'instruction à la section de réglage d'état d'affichage (114) de ne pas modifier l'état d'affichage de l'image d'affichage lorsque la valeur VD des informations de détection a atteint la seconde valeur VD2.

13. Dispositif de traitement d'images (90) tel que défini à la revendication 11,

la première section d'acquisition d'image (110) acquérant la première image correspondant à au moins une trame chaque période de cycle,

la seconde section d'acquisition d'image (111) acquérant la seconde image correspondant à au moins une trame chaque période de cycle, et

la section de mise à jour (300) réalisant le processus de mise à jour qui met à jour les informations de détection chaque période de cycle.

14. Dispositif de traitement d'images (90) tel que défini à la revendication 13,

la section de mise à jour (300) réglant une valeur VD des informations de détection à une première valeur VD1 qui correspond au temps écoulé désigné lorsque la zone d'attention a été détectée à l'intérieur de la seconde image dans une Nième (N est un nombre naturel) période de cycle, et réalisant le processus de mise à jour qui modifie séquentiellement la valeur VD des informations de détection depuis la première valeur VD1 vers une seconde valeur VD2 lorsque la zone d'attention n'a pas été détectée à l'intérieur de la seconde image dans une période de cycle qui suit la Nième période de cycle, et

la section de délivrance d'informations de commande (302) délivrant les informations de commande qui donnent l'instruction à la section de réglage d'état d'affichage (114) de modifier l'état d'affichage de l'image d'affichage jusqu'à ce que la valeur VD des informations de détection atteigne la seconde valeur VD2, et délivrant les informations de commande qui donnent l'instruction à la section de réglage d'état d'affichage (114) de ne pas modifier l'état d'affichage de l'image d'affichage lorsque la valeur VD des informations de détection a atteint la seconde valeur VD2.

15. Dispositif de traitement d'images (90) tel que défini à la revendication 14,

la section de mise à jour (300) réinitialisant la valeur VD des informations de détection à la première valeur VD1 à nouveau lorsque la zone d'attention a été détectée à l'intérieur de la seconde image dans une période de cycle qui suit la Nième période de cycle.

16. Dispositif de traitement d'images (90) tel que défini à la revendication 11,

la section de réglage d'état d'affichage (114) comprenant une section de traitement (400),

la section de traitement (400) traitant la première image, et délivrant la première image traitée en tant qu'image d'affichage lorsque les informations de commande provenant de la section de délivrance d'informations de commande (302) donnent l'instruction de modifier l'état d'affichage de l'image d'affichage, et délivrant directement la première image en tant qu'image d'affichage lorsque les informations de commande provenant de la section de délivrance d'informations de commande (302) donnent l'instruction de ne pas modifier l'état d'affichage de l'image d'affichage.

17. Dispositif de traitement d'images (90) tel que défini à la revendication 1,

la section de détection de zone d'attention (112) comprenant une section de calcul de fiabilité (205) qui calcule la fiabilité de la zone d'attention détectée par la section de détection de zone d'attention (112).

18. Dispositif de traitement d'images (90) tel que défini à la revendication 17,

la section de calcul de fiabilité (205) calculant la fiabilité sur la base d'une zone de la zone d'attention détectée par la section de détection de zone d'attention (112).

19. Dispositif de traitement d'images (90) tel que défini à la revendication 1,

la section de réglage de temps écoulé désigné (113) comprenant une section de détection de quantité de mouvement (304) qui détecte une quantité de mouvement de la première image, et

la section de réglage de temps écoulé désigné (113) réalisant le processus de réglage de temps écoulé désigné sur la base de la quantité de mouvement détectée par la section de détection de quantité de mouvement (304).

**20.** Dispositif de traitement d'images (90) tel que défini à la revendication 19, la section de réglage de temps écoulé désigné (113) réalisant le processus de réglage de temps écoulé désigné qui réduit le temps écoulé désigné lorsque la quantité de mouvement augmente.

**21.** Dispositif de traitement d'images (90) tel que défini à la revendication 19, la section de détection de quantité de mouvement (304) détectant la quantité de mouvement de la première image lorsque la zone d'attention a été détectée à l'intérieur de la seconde image.

**22.** Dispositif de traitement d'images (90) tel que défini à la revendication 19, la section de réglage de temps écoulé désigné (113) comprenant :

une section d'enregistrement d'informations de détection (301) qui enregistre des informations de détection concernant la zone d'attention ;
une section de mise à jour (300) qui réalise un processus de mise à jour qui met à jour les informations de détection sur la base du résultat de détection en provenance de la section de détection de zone d'attention (112) ; et
une section de délivrance d'informations de commande (302) qui délivre des informations de commande qui commandent le processus de réglage d'état d'affichage réalisé par la section de réglage d'état d'affichage (114) sur la base des informations de détection enregistrées dans la section d'enregistrement d'informations de détection (301), et
la section de mise à jour (300) réglant une valeur des informations de détection sur la base de la quantité de mouvement détectée par la section de détection de quantité de mouvement (304).

**23.** Dispositif de traitement d'images (90) tel que défini à la revendication 22, la section de mise à jour (300) réglant une valeur VD des informations de détection à une première valeur VD1 qui change en fonction de la quantité de mouvement détectée par la section de détection de quantité de mouvement (304) lorsque la zone d'attention a été détectée à l'intérieur de la seconde image, et réalisant le processus de mise à jour qui modifie séquentiellement la valeur VD des informations de détection depuis la première valeur VD1 vers une seconde valeur VD2 lorsque la zone d'attention n'a pas été détectée à l'intérieur de la seconde image, et la section de délivrance d'informations de commande (302) délivrant les informations de commande qui donnent l'instruction à la section de réglage d'état d'affichage (114) de modifier l'état d'affichage de l'image d'affichage jusqu'à ce que la valeur VD des informations de détection atteigne la seconde valeur VD2, et délivrant les informations de commande qui donnent l'instruction à la section de réglage d'état d'affichage (114) de ne pas modifier l'état d'affichage de l'image d'affichage lorsque la valeur VD des informations de détection a atteint la seconde valeur VD2.

**24.** Dispositif de traitement d'images (90) tel que défini à la revendication 1, la section de réglage d'état d'affichage (114) réalisant le processus de réglage d'état d'affichage qui améliore au moins un parmi un bord et une chrominance de la première image.

**25.** Dispositif de traitement d'images (90) tel que défini à la revendication 1, la bande de longueur d'onde spécifique étant plus étroite que la bande de longueur d'onde de la lumière blanche.

**26.** Dispositif de traitement d'images (90) tel que défini à la revendication 1, la première image et la seconde image étant une image *in vivo,* et la bande de longueur d'onde spécifique incluse dans l'image *in vivo* étant une bande de longueur d'onde de lumière absorbée par de l'hémoglobine dans du sang.

**27.** Dispositif de traitement d'images (90) tel que défini à la revendication 26, la bande de longueur d'onde spécifique allant de 390 à 445 nm ou de 530 à 550 nm.

**28.** Dispositif de traitement d'images (90) tel que défini à la revendication 1, la première image et la seconde image étant une image *in vivo,* et la bande de longueur d'onde spécifique incluse dans l'image *in vivo* étant une bande de longueur d'onde de fluorescence produite par une substance fluorescente.

**29.** Dispositif de traitement d'images (90) tel que défini à la revendication 28, la bande de longueur d'onde spécifique allant de 490 à 625 nm.

**30.** Dispositif de traitement d'images (90) tel que défini à la revendication 1,
la première image et la seconde image étant une image *in vivo,* et
la bande de longueur d'onde spécifique incluse dans l'image *in vivo* étant une bande de longueur d'onde de lumière infrarouge.

**31.** Dispositif de traitement d'images (90) tel que défini à la revendication 30,
la bande de longueur d'onde spécifique allant de 790 à 820 nm ou de 905 à 970 nm.

**32.** Dispositif de traitement d'images (90) tel que défini à la revendication 1,
la seconde section d'acquisition d'image (111) générant la seconde image sur la base de la première image acquise par la première section d'acquisition d'image (110).

**33.** Dispositif de traitement d'images (90) tel que défini à la revendication 32,
la seconde section d'acquisition d'image (111) comprenant une section d'extraction de signal qui extrait un signal à l'intérieur de la bande de longueur d'onde de la lumière blanche à partir de la première image acquise par la première section d'acquisition d'image (110), et
la seconde section acquisition d'image (111) générant la seconde image qui comprend un signal à l'intérieur de la bande de longueur d'onde spécifique sur la base du signal à l'intérieur de la bande de longueur d'onde de la lumière blanche extraite par la section d'extraction de signal.

**34.** Dispositif de traitement d'images (90) tel que défini à la revendication 33,
la seconde section d'acquisition d'image (111) comprenant une section de réglage de données de matrice qui règle des données de matrice pour calculer le signal à l'intérieur de la bande de longueur spécifique à partir du signal à l'intérieur de la bande de longueur d'onde de la lumière blanche, et
la seconde section d'acquisition d'image (111) calculant le signal à l'intérieur de la bande de longueur spécifique à partir du signal à l'intérieur de la bande de longueur d'onde de lumière blanche à l'aide des données de matrice réglées par la section de réglage de données de matrice pour générer la seconde image.

**35.** Programme qui amène un ordinateur à fonctionner comme :

une première section d'acquisition d'image (110) qui acquiert une première image, la première image étant une image qui possède des informations à l'intérieur d'une bande de longueur d'onde de lumière blanche ; et
une seconde section d'acquisition d'image (111) qui acquiert une seconde image, la seconde image étant une image qui possède des informations à l'intérieur d'une bande de longueur d'onde spécifique,
une section de détection de zone d'attention (112) qui détecte une zone d'attention à l'intérieur de la seconde image sur la base d'une quantité caractéristique de chaque pixel à l'intérieur de la seconde image ;
une section de réglage d'état d'affichage (114) qui réalise un processus de réglage d'état d'affichage qui règle un état d'affichage d'une image d'affichage générée sur la base de la première image ;
**caractérisé par le fait que** le programme amène l'ordinateur à fonctionner en outre comme :

une section de réglage de temps écoulé désigné (113) qui réalise un processus de réglage de temps écoulé désigné qui règle un temps écoulé désigné sur la base d'un résultat de détection pour la zone d'attention, la section de réglage d'état d'affichage (114) réalisant le processus de réglage d'état d'affichage sur la base du temps écoulé désigné qui a été réglé par la section de réglage de temps écoulé désigné (113).

# FIG. 1A

# FIG. 1B

**FIG. 2**

# FIG. 3

DISPLAY IMAGE

DISPLAY IMAGE

DISPLAY IMAGE

NORMAL LIGHT IMAGE
(FIRST IMAGE)

A1

A4
CHANGES
DISPLAY STATE
(ALERT DISPLAY)

A6
CHANGES
DISPLAY STATE
(ALERT DISPLAY)

ATTENTION AREA

SPECIAL LIGHT IMAGE
(SECOND IMAGE)

A2
ATTENTION AREA
IS DETECTED

A5

DESIGNATED
ELAPSED TIME

A3

EP 2 517 614 B1

# FIG. 4

NORMAL LIGHT IMAGE (FIRST IMAGE)

| IMN1 | IMN2 | IMN3 | IMN4 | IMN5 | IMN6 | IMN7 | IMN8 | IMN9 |
|------|------|------|------|------|------|------|------|------|
| DOES NOT CHANGE DISPLAY STATE | CHANGES DISPLAY STATE | CHANGES DISPLAY STATE | CHANGES DISPLAY STATE | CHANGES DISPLAY STATE | CHANGES DISPLAY STATE | CHANGES DISPLAY STATE | DOES NOT CHANGE DISPLAY STATE | DOES NOT CHANGE DISPLAY STATE |

B7   B6

SPECIAL LIGHT IMAGE (SECOND IMAGE)

| IMS1 | IMS2 | IMS3 | IMS4 | IMS5 | IMS6 | IMS7 | IMS8 | IMS9 |
|------|------|------|------|------|------|------|------|------|
| ATTENTION AREA IS DETECTED | ATTENTION AREA IS DETECTED | ATTENTION AREA IS NOT DETECTED | ATTENTION AREA IS NOT DETECTED | ATTENTION AREA IS NOT DETECTED | ATTENTION AREA IS NOT DETECTED | ATTENTION AREA IS NOT DETECTED | ATTENTION AREA IS NOT DETECTED | ATTENTION AREA IS NOT DETECTED |

B1    B3    B5

SETS DESIGNATED ELAPSED TIME

B2   RESETS DESIGNATED ELAPSED TIME

B4

# FIG. 5

IMAGE SIGNAL
CONTROL SIGNAL
OTHER SIGNALS

# FIG. 6A

| | | | | | |
|---|---|---|---|---|---|
| R₀₀ | G₁₀ | R₂₀ | G₃₀ | R₄₀ | G₅₀ |
| G₀₁ | B₁₁ | G₂₁ | B₃₁ | G₄₁ | B₅₁ |
| R₀₂ | G₁₂ | R₂₂ | G₃₂ | R₄₂ | G₅₂ |
| G₀₃ | B₁₃ | G₂₃ | B₃₃ | G₄₃ | B₅₃ |
| R₀₄ | G₁₄ | R₂₄ | G₃₄ | R₄₄ | G₅₄ |
| G₀₅ | B₁₅ | G₂₅ | B₃₅ | G₄₅ | B₅₅ |

# FIG. 6B

# FIG. 7A

# FIG. 7B

EP 2 517 614 B1

# FIG. 8

# FIG. 9A

| TN | TN+1 | TN+2 | TN+3 | TN+4 | TN+5 |
|---|---|---|---|---|---|
| ATTENTION AREA IS DETECTED | ATTENTION AREA IS NOT DETECTED | ATTENTION AREA IS NOT DETECTED | ATTENTION AREA IS NOT DETECTED | ATTENTION AREA IS NOT DETECTED | ATTENTION AREA IS NOT DETECTED |

C1

C3      CHANGES DISPLAY STATE

DESIGNATED ELAPSED TIME

C2

# FIG. 9B

| TN | TN+1 | TN+2 | TN+3 | TN+4 | TN+5 | TN+6 | TN+7 |
|---|---|---|---|---|---|---|---|
| ATTENTION AREA IS DETECTED | ATTENTION AREA IS DETECTED | ATTENTION AREA IS DETECTED | ATTENTION AREA IS NOT DETECTED | ATTENTION AREA IS NOT DETECTED | ATTENTION AREA IS NOT DETECTED | ATTENTION AREA IS NOT DETECTED | ATTENTION AREA IS NOT DETECTED |

C4

C6    C8      CHANGES DISPLAY STATE

SETS DESIGNATED ELAPSED TIME

C5    RESETS DESIGNATED ELAPSED TIME

C7    RESETS DESIGNATED ELAPSED TIME

C9

EP 2 517 614 B1

# FIG. 10

━━━ IMAGE SIGNAL

━━ CONTROL SIGNAL

- - - OTHER SIGNALS

EP 2 517 614 B1

112

ATTENTION AREA DETECTION SECTION

111
SECOND IMAGE ACQUISITION SECTION

200
BUFFER

201
HUE/CHROMA CALCULATION SECTION

203
THRESHOLD VALUE ROM

202
ATTENTION AREA DETERMINATION SECTION

205
RELIABILITY CALCULATION SECTION

206
AREA DETERMINATION SECTION

204
BUFFER

113
DESIGNATED ELAPSED TIME SETTING SECTION

116
CONTROL SECTION

43

# FIG. 11

# FIG. 12

—— CONTROL SIGNAL

- - - - OTHER SIGNALS

113

112

ATTENTION
AREA
DETECTION
SECTION

300

UPDATE
SECTION

DESIGNATED
ELAPSED TIME
SETTING SECTION

301

DETECTION
INFORMATION
RECORDING
SECTION

302

CONTROL
INFORMATION
OUTPUT
SECTION

114

DISPLAY
STATE
SETTING
SECTION

116

CONTROL
SECTION

EP 2 517 614 B1

# FIG. 13A

| TN | | TN+1 | | TN+2 | | TN+3 | | TN+4 | | TN+5 | | TN+6 | | TN+7 | | TN+8 |
|---|---|---|---|---|---|---|---|---|---|---|---|---|---|---|---|---|
| ATTENTION AREA IS DETECTED | × | ATTENTION AREA IS NOT DETECTED | × | ATTENTION AREA IS NOT DETECTED | × | ATTENTION AREA IS NOT DETECTED | × | ATTENTION AREA IS NOT DETECTED | × | ATTENTION AREA IS NOT DETECTED | × | ATTENTION AREA IS NOT DETECTED | × | ATTENTION AREA IS NOT DETECTED | × | ATTENTION AREA IS NOT DETECTED |

D1   D4   D5

$VD=VD1=5$   $VD=4$   $VD=3$   $VD=2$   $VD=1$   $VD=VD2=0$   $VD=0$   $VD=0$

D2   D6   D7   D8

CHANGES DISPLAY STATE          DOES NOT CHANGE DISPLAY STATE

D3          D9

# FIG. 13B

| TN | | TN+1 | | TN+2 | | TN+3 | | TN+4 | | TN+5 | | TN+6 | | TN+7 | | TN+8 |
|---|---|---|---|---|---|---|---|---|---|---|---|---|---|---|---|---|
| ATTENTION AREA IS DETECTED | × | ATTENTION AREA IS DETECTED | × | ATTENTION AREA IS NOT DETECTED | × | ATTENTION AREA IS NOT DETECTED | × | ATTENTION AREA IS NOT DETECTED | × | ATTENTION AREA IS NOT DETECTED | × | ATTENTION AREA IS NOT DETECTED | × | ATTENTION AREA IS NOT DETECTED | × | ATTENTION AREA IS NOT DETECTED |

D10   D12   D13

$VD=VD1=5$   $VD=5$   $VD=4$   $VD=3$   $VD=2$   $VD=1$   $VD=VD2=0$   $VD=0$

D11   D14   D15   D16

CHANGES DISPLAY STATE          DOES NOT CHANGE DISPLAY STATE

D17

EP 2 517 614 B1

# FIG. 14

——— IMAGE SIGNAL

——— CONTROL SIGNAL

- - - - OTHER SIGNALS

113 DESIGNATED ELAPSED TIME SETTING SECTION

114 DISPLAY STATE SETTING SECTION

110 FIRST IMAGE ACQUISITION SECTION

410 BUFFER

PROCESSING SECTION

400

401 SELECTION SECTION

402 ALERT INFORMATION ADDITION SECTION

115 DISPLAY SECTION (OUTPUT SECTION)

116 CONTROL SECTION

EP 2 517 614 B1

# FIG. 15

ATTENTION AREA
IS DETECTED

ADDS ALERT
INFORMATION

# FIG. 16

EP 2 517 614 B1

# FIG. 17

610

**MAIN BODY**

611 CPU

612 RAM

613 ROM

614 HDD

615 CD-ROM DRIVE

616 USB PORT

617 I/O INTERFACE

618 LAN INTERFACE

620 DISPLAY

630 KEYBOARD

640 MOUSE

681 PC

682 SERVER

683 PRINTER

650 MODEM

N3

# FIG. 18

```
          ┌──────────┐
          │  START   │
          └────┬─────┘
               │
    ╱──────────────────────╱ ～S1
   ╱  IMAGE SIGNAL IS INPUT ╱
  ╱──────────────────────╱
               │
        ┌──────────────┐
        │SWITCH PROCESS│ ～S2
        └──────┬───────┘
               ├───────────────────────────┐
    ┌──────────────────┐         ┌──────────────────────┐
    │ FIRST IMAGE      │ ～S3    │   SECOND IMAGE        │ ～S4
    │ ACQUISITION      │         │ ACQUISITION PROCESS   │
    │ PROCESS          │         └───────────┬──────────┘
    └────────┬─────────┘                     │
             │                   ┌──────────────────────┐
             │                   │  ATTENTION AREA      │ ～S5
             │                   │  DETECTION PROCESS   │
             │                   └───────────┬──────────┘
             │                   ┌──────────────────────┐
             │                   │ DESIGNATED ELAPSED   │ ～S6
             │◄──────────────────│ TIME SETTING PROCESS │
             │                   └──────────────────────┘
    ┌──────────────────┐
    │  DISPLAY STATE   │ ～S7
    │ SETTING PROCESS  │
    └────────┬─────────┘
             │
    ╱──────────────────────╱ ～S8
   ╱  OUTPUTS DISPLAY IMAGE ╱
  ╱──────────────────────╱
             │
           S9╱╲
          ╱      ╲
         ╱  HAVE   ╲   No
        ╱ ALL SIGNALS╲────────►
        ╲ PROCESSED? ╱
         ╲         ╱
          ╲      ╱
            ╲  ╱
           Yes│
        ┌──────────┐
        │   END    │
        └──────────┘
```

# FIG. 19

```
ATTENTION AREA
DETECTION PROCESS
        │
        ▼
HUE/CHROMA CALCULATION          S20
PROCESS
        │
        ▼
ATTENTION AREA                  S21
DETERMINATION PROCESS
        │
        ▼
RELIABILITY CALCULATION         S22
PROCESS (AREA
DETERMINATION PROCESS)
        │
        ▼
OUTPUTS DETECTION RESULT        S23
        │
        ▼
      END
```

## FIG. 20

```
┌─────────────────────────────┐
│  DESIGNATED ELAPSED         │
│  TIME SETTING PROCESS       │
└─────────────────────────────┘
              │
              ▼
      ╱─────────────────────╲
     ╱  DETECTION INFORMATION ╲────── S30
     ╲     IS INPUT           ╱
      ╲─────────────────────╱
              │
              ▼
    ┌─────────────────────┐
    │   UPDATE PROCESS    │────── S31
    └─────────────────────┘
              │
              ▼
    ┌─────────────────────┐
    │ CONTROL INFORMATION │────── S32
    │  SETTING PROCESS    │
    └─────────────────────┘
              │
              ▼
      ╱─────────────────────╲
     ╱      OUTPUTS          ╲────── S33
     ╲  CONTROL INFORMATION  ╱
      ╲─────────────────────╱
              │
              ▼
          ( END )
```

## FIG. 21

```
┌─────────────────────────────┐
│   DISPLAY STATE             │
│   SETTING PROCESS           │
└─────────────────────────────┘
              │
              ▼
      ╱─────────────────────╲
     ╱  CONTROL INFORMATION   ╲────── S40
     ╲     IS INPUT           ╱
      ╲─────────────────────╱
              │
              ▼
         ◇─────────◇  S41
        ╱  CHANGES  ╲        No
        ◇ DISPLAY   ◇──────────┐
        ╲ STATE?   ╱           │
         ◇─────────◇           │
              │ Yes            │
              ▼                │
    ┌─────────────────────┐    │
    │  ALERT INFORMATION  │─── S42
    │  ADDITION PROCESS   │    │
    └─────────────────────┘    │
              │◄───────────────┘
              ▼
          ( END )
```

# FIG. 22

EP 2 517 614 B1

——— IMAGE SIGNAL

——— CONTROL SIGNAL

- - - - OTHER SIGNALS

90

**IMAGE PROCESSING DEVICE**

101

104
GAIN AMPLI-FIER

105
A/D CONVERSION SECTION

106
BUFFER

109
SWITCH SECTION

110
FIRST IMAGE ACQUISITION SECTION

501
DISPLAY STATE SETTING SECTION

115
DISPLAY SECTION (OUTPUT SECTION)

107
WB SECTION

111
SECOND IMAGE ACQUISITION SECTION

100

102

108
PHOTO-METRICAL EVALUATION SECTION

112
ATTENTION AREA DETECTION SECTION

500
DESIGNATED ELAPSED TIME SETTING SECTION

103

116
CONTROL SECTION

117
EXTERNAL I/F SECTION

54

# FIG. 23

EP 2 517 614 B1

FIG. 24

# FIG. 25

# FIG. 26

IMAGE SIGNAL
CONTROL SIGNAL
- - - - OTHER SIGNALS

500
DESIGNATED
ELAPSED TIME
SETTING
SECTION

501
DISPLAY STATE SETTING SECTION

410
BUFFER

401
SELECTION
SECTION

PROCESS SECTION

398

FIRST IMAGE
ACQUISITION
SECTION

110

115
DISPLAY
SECTION
(OUTPUT
SECTION)

403
LUMINANCE/COLOR
DIFFERENCE
SEPARATION
SECTION

404
EDGE
ENHANCEMENT
SECTION

406
LUMINANCE/
COLOR
DIFFERENCE
BLENDING SECTION

405
CHROMA
ENHANCEMENT
SECTION

116
CONTROL
SECTION

EP 2 517 614 B1

# FIG. 27

━━━ IMAGE SIGNAL

── CONTROL SIGNAL

---- OTHER SIGNALS

90 — IMAGE PROCESSING DEVICE

F1 (NORMAL LIGHT IMAGE FILTER)

F2 (SPECIAL LIGHT IMAGE FILTER)

100

550

551

102

104 GAIN AMPLIFIER

105 A/D CONVERSION SECTION

106 BUFFER

107 WB SECTION

108 PHOTO-METRICAL EVALUATION SECTION

552 SWITCH SECTION

110 FIRST IMAGE ACQUISITION SECTION

111 SECOND IMAGE ACQUISITION SECTION

112 ATTENTION AREA DETECTION SECTION

113 DESIGNATED ELAPSED TIME SETTING SECTION

114 DISPLAY STATE SETTING SECTION

115 DISPLAY SECTION (OUTPUT SECTION)

116 CONTROL SECTION

117 EXTERNAL I/F SECTION

EP 2 517 614 B1

59

# FIG. 28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 63122421 A
- JP 2004321244 A
- US 20050059894 A1
- JP 2002095635 A